(19) Europäisches Patentamt / European Patent Office / Office européen des brevets

(11) **EP 1 659 171 B1**

(12) **EUROPEAN PATENT SPECIFICATION**


(45) Date of publication and mention of the grant of the patent: **06.08.2008 Bulletin 2008/32**

(21) Application number: **03818540.1**

(22) Date of filing: **29.08.2003**

(51) Int Cl.: ***C02F 3/34*** *(2006.01)*

(86) International application number: **PCT/JP2003/011008**

(87) International publication number: **WO 2005/023997 (17.03.2005 Gazette 2005/11)**


(54) **NOVEL MICROORGANISM AND METHOD OF TREATING ORGANIC SOLID MATTERS WITH THE USE OF THE MICROORGANISM**

NEUER MIKROORGANISMUS UND VERFAHREN ZUR BEHANDLUNG ORGANISCHER FESTSTOFFE UNTER VERWENDUNG DES MIKROORGANISMUS

NOUVEAU MICRO-ORGANISME ET PROCEDE DE TRAITEMENT DE MATIERES SOLIDES ORGANIQUES UTILISANT CE MICRO-ORGANISME


(84) Designated Contracting States: **AT BE BG CH CY CZ DE DK EE ES FI FR GB GR HU IE IT LI LU MC NL PT RO SE SI SK TR**

(30) Priority: **27.08.2003 JP 2003209106**

(43) Date of publication of application: **24.05.2006 Bulletin 2006/21**



(73) Proprietor: **KOBELCO ECO-SOLUTIONS CO., LTD.**
**Kobe-shi,**
**Hyogo 651-0072 (JP)**

(72) Inventors:
- **AKASHI, Akira**
  **Kobe-shi, Hyogo 655-0043 (JP)**
- **TAKATA, Kazutaka**
  **Kobe-shi, Hyogo 655-0039 (JP)**
- **HASEGAWA, Susumu**
  **Kobe-shi, Hyogo 651-2242 (JP)**

(74) Representative: **Wablat, Wolfgang**
**Patentanwalt**
**Dr. Dr. W. Wablat**
**Potsdamer Chaussee 48**
**14129 Berlin (DE)**



(56) References cited:
**JP-A- 9 234 060**
**JP-A- 2000 139 449**
**JP-A- 2000 167 596**
**JP-A- 2002 125 657**
**JP-A- 2003 062 559**

- **SUNG M H ET AL: "Geobacillus toebii sp. nov., a novel thermophilic bacterium isolated from hay compost." INTERNATIONAL JOURNAL OF SYSTEMATIC AND EVOLUTIONARY MICROBIOLOGY. NOV 2002, vol. 52, no. Pt 6, November 2002 (2002-11), pages 2251-2255, XP002404563 ISSN: 1466-5026**
- **NAZINA T N ET AL: "Taxonomic study of aerobic thermophilic bacilli: descriptions of Geobacillus subterraneus gen. nov., sp. nov. and Geobacillus uzenensis sp. nov. from petroleum reservoirs and transfer of Bacillus stearothermophilus, Bacillus thermocatenulatus, Bacillus thermoleovorans, Bacillus kaustophilus, Bacillu" INTERNATIONAL JOURNAL OF SYSTEMATIC AND EVOLUTIONARY MICROBIOLOGY. MAR 2001, vol. 51, no. Pt 2, March 2001 (2001-03), pages 433-446, XP002404564 ISSN: 1466-5026**
- **MARCHANT ROGER ET AL: "The frequency and characteristics of highly thermophilic bacteria in cool soil environments." ENVIRONMENTAL MICROBIOLOGY. OCT 2002, vol. 4, no. 10, October 2002 (2002-10), pages 595-602, XP002404635 ISSN: 1462-2912**
- **DONG, X. ET AL.: 'Phylogenetic relationships between Bacillus species and related genera inferred from comparison of 3' end 16S rDNA and 5' end 16S-23S ITS nucleotide sequences' INT.J.SYST.EVOL.MICROBIOL. vol. 53, no. 3, May 2003, pages 695 - 704, XP002903460**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 1 659 171 B1

- OBOJSKA, A. ET AL.: 'Organophosphonate utilization by the thermophile Geobacillus caldoxylosilyticus T20' APPL.ENVIRON.MICROBIOL. vol. 68, no. 4, April 2002, pages 2081 - 2084, XP002903461
- MAUGERI, T.L. ET AL.: 'Three novel halotolerant and thermophilic Geobacillus strains from shallow marine vents' SYST.APPL.MICROBIOL. vol. 25, no. 3, October 2002, pages 450 - 455, XP002903462

SEQUENCE LISTING

<110> SHINKO PANTEC CO.,LTD
<120> NOVEL MICROORGANISM AND PROCESS FOR TREATMENT OF ORGANIC SOLID MATTER USING THE MICROORGANISM
<130> Fuj-22103 EP
<140> EP03818540.1
<141> 2003-08-29
<150> JP2003209106
<151> 2003-08-27
<160> 6

<210> 1
<211> 1495
<212> DNA
<213> Geobacillus sp.
<220>
<223> /note = 16SrRNA gene of Geobacillus sp.SPT4(FERM BP-08452)

<400> 1

```
gaacgctggc ggcgtgccta atacatgcaa gtcgagcgga ccggacagga gcttgctctt     60
gttcggttag cggcggacgg gtgagtaaca cgtgggcaac ctacccgtaa gaccgggata    120
actccgggaa accggagcta ataccggata acaccgaaga ccgcatggtc ttcggttgaa    180
aggcggcttt ggctgtcact tacggatggg cccgcggcgc attagctagt tggtgaggta    240
acggctcacc aaggcgacga tgcgtagccg gcctgagagg gtgaccggcc acactgggac    300
tgagacacgg cccagactcc tacgggaggc agcagtaggg aatcttccgc aatggacgaa    360
agtctgacgg agcgacgccg cgtgagcgaa gaaggtcttc ggatcgtaaa gctctgttgt    420
tagggaagaa gaagtaccgt tcgaataggg cggtacggtg acggtaccta acgagaaagc    480
cccggctaac tacgtgccag cagccgcggt aatacgtagg ggcgagcgtt gtccggaatt    540
attgggcgta aagcgcgcgc aggcggtccc ttaagtctga tgtgaaagcc cacggctcaa    600
ccgtggaggg tcattggaaa ctgggggact tgagtgcaga agaggagagc ggaattccac    660
gtgtagcggt gaaatgcgta gagatgtgga ggaacaccag tggcgaaggc ggctctctgg    720
tctgtaactg acgctgaggc gcgaaagcgt ggggagcaaa caggattaga taccctggta    780
gtccacgccg taaacgatga gtgctaagtg ttagaggggt caaacccttt agtgctgcag    840
ctaacgcgtt aagcactccg cctggggagt acggccgcaa ggctgaaact caaaggaatt    900
gacgggggcc cgcacaagcg gtggagcatg tggtttaatt cgaagcaacg cgaagaacct    960
taccaggtct tgacatcccc tgacaaccct agagataggg cgttcccct tcgggggac    1020
agggtgacag gtggtgcatg gttgtcgtca gctcgtgtcg tgagatgttg ggttaagtcc   1080
cgcaacgagc gcaaccctcg accttagttg ccagcattca gttgggcact ctaaggtgac   1140
tgccgatgac aaatcggagg aaggtgggga tgacgtcaaa tcatcatgcc ccttatgacc   1200
tgggctacac acgtgctaca atgggcggta caaagggctg cgaacccgcg agggggagcg   1260
aatcccaaaa agccgctctc agttcggatt gcaggctgca actcgcctgc atgaagccgg   1320
aatcgctagt aatcgcggat cagcatgccg cggtgaatac gttcccgggc cttgtacaca   1380
ccgcccgtca caccacgaga gcttgcaaca cccgaagtcg gtgaggtaac cctttcggga   1440
gccagccgcc gaaggtgggg caagtgattg ggtgaagtc gtaacaggt agcca          1495
```

<210> 2
<211> 1498

<212> DNA
<213> Geobacillus sp.
<220>
<223> /note = 16SrRNA gene of Geobacillus sp.SPT5(FERM BP-08453)

<400> 2

```
agaacgctgg cggcgtgcct aatacatgca agtcgagcgg actgaatggg agcttgctct      60
tgttcggtca gcggcggacg ggtgagtaac acgtgggcaa cctgcccgca agaccgggat     120
aactccggga aaccggagct aataccggat aacaccgaag accgcatggt ctttggttga     180
aaggcggcgc aagctgccac ttgcggatgg gcccgcggcg cattagctag ttggtgaggt     240
aacggctcac caaggcgacg atgcgtagcc ggcctgagag ggtgaccggc cacactggga     300
ctgagacacg gcccagactc ctacgggagg cagcagtagg gaatcttccg caatgggcga     360
aagcctgacg gagcgacgcc gcgtgagcga agaaggcctt cgggtcgtaa agctctgttg     420
tgagggacga aggagcgccg tttgaagaag gcggcgcggt gacggtacct cacgaggaag     480
ccccggctaa ctacgtgcca gcagccgcgg taatacgtag ggcgagcgt tgtccggaat     540
tattgggcgt aaagcgcgcg caggcggttc cttaagtctg atgtgaaagc ccacggctca     600
accgtggagg gtcattggaa actgggggac ttgagtgcag gagaggagag cggaattcca     660
cgtgtagcgg tgaaatgcgt agagatgtgg aggaacacca gtggcgaagg cggctctctg     720

gcctgcaact gacgctgagg cgcgaaagcg tggggagcaa acaggattag ataccctggt     780
agtccacgcc gtaaacgatg agtgctaagt gttagagggg tcacaccctt tagtgctgca     840
gctaacgcga taagcactcc gcctggggag tacggccgca aggctgaaac tcaaaggaat     900
tgacgggggc ccgcacaagc ggtggagcat gtggtttaat tcgaagcaac gcgaagaacc     960
ttaccaggtc ttgacatccc ctgacaaccc aagagattgg gcgttccccc ttcgggggga    1020
cagggtgaca ggtggtgcat ggttgtcgtc agctcgtgtc gtgagatgtt gggttaagtc    1080
ccgcaacgag cgcaaccctt cgcctctagt tgccagcatt cggttgggca ctctagaggg    1140
actgccggcg acaagtcgga ggaaggtggg gatgacgtca aatcatcatg ccccttatga    1200
cctgggctac acacgtgcta caatggcgg tacaaagggc tgcgaacccg cgagggggag    1260
cgaatcccaa aaagccgctc tcagttcgga ttgcaggctg caactcgcct gcatgaagcc    1320
ggaatcgcta gtaatcgcgg atcagcatgc cgcggtgaat acgttcccgg gccttgtaca    1380
caccgcccgt cacaccacga gagcttgcaa cacccgaagt cggtgaggca acccgtttcg    1440
ggagccagcc gccgaaggtg gggcaagtga ttggggtgaa gtcgtaacag ggtagcca      1498
```

<210> 3
<211> 1495
<212> DNA
<213> Geobacillus sp.
<220>
<223> /note = 16SrRNA gene of Geobacillus sp.SPT6(FERM BP-08454)

<400> 3

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggggact tgagtgcaga agaggagagc ggaattccac    660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccccct tcgggggac   1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg gggtgaagtc gtaacagggt agcca         1495
```

<210> 4
<211> 1498
<212> DNA
<213> Geobacillus sp.
<220>
<223> /note = 16SrRNA gene of Geobacillus sp.SPT7(FERM BP-08455)

<400> 4

```
gagaacgctg gcggcgtgcc taatacatgc aagtcgagcg gaccaaatcg gagcttgctc      60
tgatttggtc agcggcggac gggtgagtaa cacgtgggca acctgcccgc aagaccggga     120
taactccggg aaaccggagc taataccgga taacaccgaa gaccgcatgg tctttggttg     180
aaaggcggcc tttggctgtc acttgcggat gggcccgcgg cgcattagct agttggtgag     240
gtaacggctc accaaggcga cgatgcgtag ccggcctgag agggtgaccg gccacactgg     300
gactgagaca cggcccagac tcctacggga ggcagcagta gggaatcttc cgcaatgggc     360
gaaagcctga cggagcgacg ccgcgtgagc gaagaaggcc ttcgggtcgt aaagctctgt     420
tgtgagggac gaaggagcgc cgttcgaaga gggcggcgcg gtgacggtac ctcacgagga     480
agccccggct aactacgtgc cagcagccgc ggtaatacgt aggggcgagc gttgtccgga     540
```

```
attattgggc gtaaagcgcg cgcaggcggt tccttaagtc tgatgtgaaa gcccacggct     600
caaccgtgga gggtcattgg aaactggggg acttgagtgc aggagaggag agcggaattc     660
cacgtgtagc ggtgaaatgc gtagagatgt ggaggaacac cagtggcgaa ggcggctctc     720
tggcctgcaa ctgacgctga ggcgcgaaag cntggggagc aaacaggatt agataccctg     780
gtagtccacg ccgtaaacga tgagtgctaa gtgttagagg ggtcacaccc tttagtgctg     840
cagctaacgc gataagcact ccgcctgggg agtacggccg caaggctgaa actcaaagga     900
attgacgggg gcccgcacaa gcggtggagc atgtggttta attcgaagca acgcgaagaa     960
ccttaccagg tcttgacatc ccctgacaac ccaagagatt gggcgttccc cttcgggggg    1020
gacagggtga caggtggtgc atggttgtcg tcagctcgtg tcgtgagatg ttgggttaag    1080
tcccgcaacg agcgcaaccc tcgcctctag ttgccagcac gaangtgggc actctagagg    1140
gactgccggc gacaagtcgg aggaaggtgg ggatgacgtc aaatcatcat gccccttatg    1200
acctgggcta cacacgtgct acaatgggcg gtacaaaggg ctgcgaaccc gcgaggggga    1260
gcgaatccca aaaagccgct ctcagttcgg attgcaggct gcaactcgcc tgcatgaagc    1320
cggaatcgct agtaatcgcg gatcagcatg ccgcggtgaa tacgttcccg ggccttgtac    1380
acaccgcccg tcacaccacg agagcttgca acacccgaag tcggtgnggt aacccttacg    1440
ggagccagcc gccgaaggtg ggcaagtga ttggggtgaa gtcgtaacag ggtagcca       1498
```

<210> 5
<211> 20
<212> DNA
<213> Artificial Sequence
<220>
<223> Synthetic oligonucleotide 27f

<400> 5
agagtttgat cctgcctcag 20

<210> 6
<211> 19
<212> DNA
<213> Artificial Sequence
<220>
<223> Synthetic oligonucleotide 1492r

<400> 6
ggctaccttg ttacgactt 19

SEQUENCE LISTING

<110> SHINKO PANTEC CO.,LTD
<120> NOVEL MICROORGANISM AND PROCESS FOR TREATMENT OF ORGANIC SOLID MATTER USING THE MICROORGANISM
<130> Fuj-22103 EP
<140> EP03818540.1
<141> 2003-08-29
<150> JP2003209106
<151> 2003-08-27
<160> 6

<210> 1
<211> 1495
<212> DNA
<213> Geobacillus sp.
<220>
<223> /note = 16SrRNA gene of Geobacillus sp.SPT4(FERM BP-08452)

<400> 1

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggggact tgagtgcaga agaggagagc ggaattccac    660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccccct tcgggggac   1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg ggtgaagtc gtaacagggt agcca          1495
```

<210> 2
<211> 1498
<212> DNA
<213> Geobacillus sp.
<220>
<223> /note = 16SrRNA gene of Geobacillus sp. SPT5 (FERM BP-08453)

<400> 2

```
agaacgctgg cggcgtgcct aatacatgca agtcgagcgg actgaatggg agcttgctct      60
tgttcggtca gcggcggacg ggtgagtaac acgtgggcaa cctgcccgca agaccgggat     120
aactccggga aaccggagct aataccggat aacaccgaag accgcatggt ctttggttga     180
aaggcggcgc aagctgccac ttgcggatgg gcccgcggcg cattagctag ttggtgaggt     240
aacggctcac caaggcgacg atgcgtagcc ggcctgagag ggtgaccggc cacactggga     300
ctgagacacg gcccagactc ctacgggagg cagcagtagg gaatcttccg caatgggcga     360
aagcctgacg gagcgacgcc gcgtgagcga agaaggcctt cgggtcgtaa agctctgttg     420
tgagggacga aggagcgccg tttgaagaag gcggcgcggt gacggtacct cacgaggaag     480
ccccggctaa ctacgtgcca gcagccgcgg taatacgtag ggcgagcgt tgtccggaat      540
tattgggcgt aaagcgcgcg caggcggttc cttaagtctg atgtgaaagc ccacggctca     600
accgtggagg gtcattggaa actgggggac ttgagtgcag gagaggagag cggaattcca     660
cgtgtagcgg tgaaatgcgt agagatgtgg aggaacacca gtggcgaagg cggctctctg     720
```

```
gcctgcaact gacgctgagg cgcgaaagcg tggggagcaa acaggattag ataccctggt    780
agtccacgcc gtaaacgatg agtgctaagt gttagagggg tcacaccctt tagtgctgca    840
gctaacgcga taagcactcc gcctggggag tacggccgca aggctgaaac tcaaaggaat    900
tgacgggggc ccgcacaagc ggtggagcat gtggtttaat tcgaagcaac gcgaagaacc    960
ttaccaggtc ttgacatccc ctgacaaccc aagagattgg gcgttccccc ttcgggggga   1020
cagggtgaca ggtggtgcat ggttgtcgtc agctcgtgtc gtgagatgtt gggttaagtc   1080
ccgcaacgag cgcaaccctt cgcctctagt tgccagcatt cggttgggca ctctagaggg   1140
actgccggcg acaagtcgga ggaaggtggg gatgacgtca aatcatcatg ccccttatga   1200
cctgggctac acacgtgcta caatgggcgg tacaaagggc tgcgaacccg cgaggggag    1260
cgaatcccaa aaagccgctc tcagttcgga ttgcaggctg caactcgcct gcatgaagcc   1320
ggaatcgcta gtaatcgcgg atcagcatgc cgcggtgaat acgttcccgg gccttgtaca   1380
caccgcccgt cacaccacga gagcttgcaa cacccgaagt cggtgaggca acccgtttcg   1440
ggagccagcc gccgaaggtg gggcaagtga ttggggtgaa gtcgtaacag ggtagcca     1498
```

<210> 3
<211> 1495
<212> DNA
<213> Geobacillus sp.
<220>
<223> /note = 16SrRNA gene of Geobacillus sp.SPT6(FERM BP-08454)

<400> 3

```
gaacgctggc ggcgtgccta atacatgcaa gtcgagcgga ccggacagga gcttgctctt     60
gttcggttag cggcggacgg gtgagtaaca cgtgggcaac ctacccgtaa gaccgggata    120
actccgggaa accggagcta ataccggata acaccgaaga ccgcatggtc ttcggttgaa    180
aggcggcttt ggctgtcact tacggatggg cccgcggcgc attagctagt tggtgaggta    240
acggctcacc aaggcgacga tgcgtagccg gcctgagagg gtgaccggcc acactgggac    300
tgagacacgg cccagactcc tacgggaggc agcagtaggg aatcttccgc aatggacgaa    360
agtctgacgg agcgacgccg cgtgagcgaa gaaggtcttc ggatcgtaaa gctctgttgt    420
tagggaagaa gaagtaccgt tcgaataggg cggtacggtg acggtaccta acgagaaagc    480
cccggctaac tacgtgccag cagccgcggt aatacgtagg ggcgagcgtt gtccggaatt    540
attgggcgta aagcgcgcgc aggcggtccc ttaagtctga tgtgaaagcc cacggctcaa    600
ccgtggaggg tcattggaaa ctgggggact tgagtgcaga agaggagagc ggaattccac    660
gtgtagcggt gaaatgcgta gagatgtgga ggaacaccag tggcgaaggc ggctctctgg    720
tctgtaactg acgctgaggc gcgaaagcgt ggggagcaaa caggattaga taccctggta    780
gtccacgccg taaacgatga gtgctaagtg ttagaggggt caaacccttt agtgctgcag    840
ctaacgcgtt aagcactccg cctggggagt acggccgcaa ggctgaaact caaaggaatt    900
gacgggggcc cgcacaagcg gtggagcatg tggtttaatt cgaagcaacg cgaagaacct    960
taccaggtct tgacatcccc tgacaaccct agagataggg cgttccccct tcgggggac    1020
agggtgacag gtggtgcatg gttgtcgtca gctcgtgtcg tgagatgttg ggttaagtcc   1080
cgcaacgagc gcaaccctcg accttagttg ccagcattca gttgggcact ctaaggtgac   1140
tgccgatgac aaatcggagg aaggtgggga tgacgtcaaa tcatcatgcc ccttatgacc   1200
tgggctacac acgtgctaca atgggcggta caaagggctg cgaacccgcg aggggagcg    1260
aatcccaaaa agccgctctc agttcggatt gcaggctgca actcgcctgc atgaagccgg   1320
aatcgctagt aatcgcggat cagcatgccg cggtgaatac gttcccgggc cttgtacaca   1380
ccgcccgtca caccacgaga gcttgcaaca cccgaagtcg gtgaggtaac cctttcggga   1440
gccagccgcc gaaggtgggg caagtgattg ggtgaagtc gtaacagggt agcca         1495
```

<210> 4
<211> 1498
<212> DNA
<213> Geobacillus sp.
<220>
<223> /note = 16SrRNA gene of Geobacillus sp.SPT7(FERM BP-08455)

<400> 4

```
gagaacgctg gcggcgtgcc taatacatgc aagtcgagcg gaccaaatcg gagcttgctc      60
tgatttggtc agcggcggac gggtgagtaa cacgtgggca acctgcccgc aagaccggga     120
taactccggg aaaccggagc taataccgga taacaccgaa gaccgcatgg tctttggttg     180
aaaggcggcc tttggctgtc acttgcggat gggcccgcgg cgcattagct agttggtgag     240
gtaacggctc accaaggcga cgatgcgtag ccggcctgag agggtgaccg gccacactgg     300
gactgagaca cggcccagac tcctacggga ggcagcagta gggaatcttc cgcaatgggc     360
gaaagcctga cggagcgacg ccgcgtgagc gaagaaggcc ttcgggtcgt aaagctctgt     420
tgtgagggac gaaggagcgc cgttcgaaga gggcggcgcg gtgacggtac ctcacgagga     480
agccccggct aactacgtgc cagcagccgc ggtaatacgt aggggcgagc gttgtccgga     540

attattgggc gtaaagcgcg cgcaggcggt tccttaagtc tgatgtgaaa gcccacggct     600
caaccgtgga gggtcattgg aaactggggg acttgagtgc aggagaggag agcggaattc     660
cacgtgtagc ggtgaaatgc gtagagatgt ggaggaacac cagtggcgaa ggcggctctc     720
tggcctgcaa ctgacgctga ggcgcgaaag cntggggagc aaacaggatt agataccctg     780
gtagtccacg ccgtaaacga tgagtgctaa gtgttagagg ggtcacaccc tttagtgctg     840
cagctaacgc gataagcact ccgcctgggg agtacggccg caaggctgaa actcaaagga     900
attgacgggg gcccgcacaa gcggtggagc atgtggttta attcgaagca acgcgaagaa     960
ccttaccagg tcttgacatc ccctgacaac ccaagagatt gggcgttccc ccttcggggg    1020
gacagggtga caggtggtgc atggttgtcg tcagctcgtg tcgtgagatg ttgggttaag    1080
tcccgcaacg agcgcaaccc tcgcctctag ttgccagcac gaangtgggc actctagagg    1140
gactgccggc gacaagtcgg aggaaggtgg ggatgacgtc aaatcatcat gccccttatg    1200
acctgggcta cacacgtgct acaatgggcg gtacaaaggg ctgcgaaccc gcgaggggga    1260
gcgaatccca aaaagccgct ctcagttcgg attgcaggct gcaactcgcc tgcatgaagc    1320
cggaatcgct agtaatcgcg gatcagcatg ccgcggtgaa tacgttcccg ggccttgtac    1380
acaccgcccg tcacaccacg agagcttgca acacccgaag tcggtgnggt aacccttacg    1440
ggagccagcc gccgaaggtg ggcaagtga ttggggtgaa gtcgtaacag ggtagcca       1498
```

<210> 5
<211> 20
<212> DNA
<213> Artificial Sequence
<220>
<223> Synthetic oligonucleotide 27f

<400> 5
agagtttgat cctgcctcag 20

<210> 6
<211> 19
<212> DNA
<213> Artificial Sequence
<220>
<223> Synthetic oligonucleotide 1492r

<400> 6
ggctaccttg ttacgactt 19

SEQUENCE LISTING

<110> SHINKO PANTEC CO., LTD
<120> NOVEL MICROORGANISM AND PROCESS FOR TREATMENT OF ORGANIC SOLID MATTER USING THE MICROORGANISM
<130> Fuj-22103 EP
<140> EP03818540.1
<141> 2003-08-29
<150> JP2003209106

<151> 2003-08-27
<160> 6

<210> 1
<211> 1495
<212> DNA
<213> Geobacillus sp.
<220>
<223> /note = 16SrRNA gene of Geobacillus sp.SPT4(FERM BP-08452)

<400> 1

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggggact tgagtgcaga agaggagagc ggaattccac     660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttccccct tcggggggac    1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg ggtgaagtc gtaacagggt agcca          1495
```

<210> 2
<211> 1498
<212> DNA
<213> Geobacillus sp.
<220>
<223> /note = 16SrRNA gene of Geobacillus sp.SPT5(FERM BP-08453)

<400> 2

```
agaacgctgg cggcgtgcct aatacatgca agtcgagcgg actgaatggg agcttgctct      60
tgttcggtca gcggcggacg ggtgagtaac acgtgggcaa cctgcccgca agaccgggat     120
aactccggga aaccggagct aataccggat aacaccgaag accgcatggt ctttggttga     180
aaggcggcgc aagctgccac ttgcggatgg gcccgcggcg cattagctag ttggtgaggt     240
aacggctcac caaggcgacg atgcgtagcc ggcctgagag ggtgaccggc cacactggga     300
ctgagacacg gcccagactc ctacgggagg cagcagtagg gaatcttccg caatgggcga     360
aagcctgacg gagcgacgcc gcgtgagcga agaaggcctt cgggtcgtaa agctctgttg     420
tgagggacga aggagcgccg tttgaagaag gcggcgcggt gacggtacct cacgaggaag     480
ccccggctaa ctacgtgcca gcagccgcgg taatacgtag gggcgagcgt tgtccggaat     540
tattgggcgt aaagcgcgcg caggcggttc cttaagtctg atgtgaaagc ccacggctca     600
accgtggagg gtcattggaa actggggggac ttgagtgcag gagaggagag cggaattcca     660
cgtgtagcgg tgaaatgcgt agagatgtgg aggaacacca gtggcgaagg cggctctctg     720
```

gcctgcaact gacgctgagg cgcgaaagcg tggggagcaa acaggattag ataccctggt 780
agtccacgcc gtaaacgatg agtgctaagt gttagagggg tcacaccctt tagtgctgca 840
gctaacgcga taagcactcc gcctggggag tacggccgca aggctgaaac tcaaaggaat 900
tgacgggggc ccgcacaagc ggtggagcat gtggtttaat tcgaagcaac gcgaagaacc 960
ttaccaggtc ttgacatccc ctgacaaccc aagagattgg gcgttccccc ttcgggggga 1020
cagggtgaca ggtggtgcat ggttgtcgtc agctcgtgtc gtgagatgtt gggttaagtc 1080
ccgcaacgag cgcaaccctt cgcctctagt tgccagcatt cggttgggca ctctagaggg 1140
actgccggcg acaagtcgga ggaaggtggg gatgacgtca aatcatcatg ccccttatga 1200
cctgggctac acacgtgcta caatgggcgg tacaaagggc tgcgaacccg cgagggggag 1260
cgaatcccaa aaagccgctc tcagttcgga ttgcaggctg caactcgcct gcatgaagcc 1320
ggaatcgcta gtaatcgcgg atcagcatgc cgcggtgaat acgttcccgg gccttgtaca 1380
caccgcccgt cacaccacga gagcttgcaa cacccgaagt cggtgaggca acccgtttcg 1440
ggagccagcc gccgaaggtg gggcaagtga ttggggtgaa gtcgtaacag ggtagcca 1498

<210> 3
<211> 1495
<212> DNA
<213> Geobacillus sp.
<220>
<223> /note = 16SrRNA gene of Geobacillus sp.SPT6 (FERM BP-08454)

<400> 3

gaacgctggc ggcgtgccta atacatgcaa gtcgagcgga ccggacagga gcttgctctt 60
gttcggttag cggcggacgg gtgagtaaca cgtgggcaac ctacccgtaa gaccgggata 120
actccgggaa accggagcta ataccggata acaccgaaga ccgcatggtc ttcggttgaa 180
aggcggcttt ggctgtcact tacggatggg cccgcggcgc attagctagt tggtgaggta 240
acggctcacc aaggcgacga tgcgtagccg gcctgagagg gtgaccggcc acactgggac 300
tgagacacgg cccagactcc tacgggaggc agcagtaggg aatcttccgc aatggacgaa 360
agtctgacgg agcgacgccg cgtgagcgaa gaaggtcttc ggatcgtaaa gctctgttgt 420
tagggaagaa gaagtaccgt tcgaataggg cggtacggtg acggtaccta acgagaaagc 480
cccggctaac tacgtgccag cagccgcggt aatacgtagg ggcgagcgtt gtccggaatt 540
attgggcgta aagcgcgcgc aggcggtccc ttaagtctga tgtgaaagcc cacggctcaa 600
ccgtggaggg tcattggaaa ctgggggact tgagtgcaga agaggagagc ggaattccac 660
gtgtagcggt gaaatgcgta gagatgtgga ggaacaccag tggcgaaggc ggctctctgg 720
tctgtaactg acgctgaggc gcgaaagcgt ggggagcaaa caggattaga taccctggta 780
gtccacgccg taaacgatga gtgctaagtg ttagaggggt caaacccttt agtgctgcag 840
ctaacgcgtt aagcactccg cctggggagt acggccgcaa ggctgaaact caaaggaatt 900
gacgggggcc cgcacaagcg gtggagcatg tggtttaatt cgaagcaacg cgaagaacct 960
taccaggtct tgacatcccc tgacaaccct agagataggg cgttccccct tcgggggac 1020
agggtgacag gtggtgcatg gttgtcgtca gctcgtgtcg tgagatgttg ggttaagtcc 1080
cgcaacgagc gcaaccctcg accttagttg ccagcattca gttgggcact ctaaggtgac 1140
tgccgatgac aaatcggagg aaggtgggga tgacgtcaaa tcatcatgcc ccttatgacc 1200
tgggctacac acgtgctaca atgggcggta caaagggctg cgaacccgcg aggggagcg 1260
aatcccaaaa agccgctctc agttcggatt gcaggctgca actcgcctgc atgaagccgg 1320
aatcgctagt aatcgcggat cagcatgccg cggtgaatac gttcccgggc cttgtacaca 1380
ccgcccgtca caccacgaga gcttgcaaca cccgaagtcg gtgaggtaac cctttcggga 1440
gccagccgcc gaaggtgggg caagtgattg gggtgaagtc gtaacagggt agcca 1495

<210> 4
<211> 1498
<212> DNA
<213> Geobacillus sp.
<220>
<223> /note = 16SrRNA gene of Geobacillus sp. SPT7 (FERM BP-08455)

<400> 4

gagaacgctg gcggcgtgcc taatacatgc aagtcgagcg gaccaaatcg gagcttgctc 60
tgatttggtc agcggcggac gggtgagtaa cacgtgggca acctgcccgc aagaccggga 120
taactccggg aaaccggagc taataccgga taacaccgaa gaccgcatgg tctttggttg 180
aaaggcggcc tttggctgtc acttgcggat gggcccgcgg cgcattagct agttggtgag 240
gtaacggctc accaaggcga cgatgcgtag ccggcctgag agggtgaccg gccacactgg 300
gactgagaca cggcccagac tcctacggga ggcagcagta gggaatcttc cgcaatgggc 360
gaaagcctga cggagcgacg ccgcgtgagc gaagaaggcc ttcgggtcgt aaagctctgt 420
tgtgagggac gaaggagcgc cgttcgaaga gggcggcgcg gtgacggtac ctcacgagga 480
agccccggct aactacgtgc cagcagccgc ggtaatacgt aggggcgagc gttgtccgga 540

```
attattgggc gtaaagcgcg cgcaggcggt tccttaagtc tgatgtgaaa gcccacggct    600
caaccgtgga gggtcattgg aaactggggg acttgagtgc aggagaggag agcggaattc    660
cacgtgtagc ggtgaaatgc gtagagatgt ggaggaacac cagtggcgaa ggcggctctc    720
tggcctgcaa ctgacgctga ggcgcgaaag cntggggagc aaacaggatt agataccctg    780
gtagtccacg ccgtaaacga tgagtgctaa gtgttagagg ggtcacaccc tttagtgctg    840
cagctaacgc gataagcact ccgcctgggg agtacggccg caaggctgaa actcaaagga    900
attgacgggg gcccgcacaa gcggtggagc atgtggttta attcgaagca acgcgaagaa    960
ccttaccagg tcttgacatc ccctgacaac ccaagagatt gggcgttccc ccttcggggg   1020
gacagggtga caggtggtgc atggttgtcg tcagctcgtg tcgtgagatg ttgggttaag   1080
tcccgcaacg agcgcaaccc tcgcctctag ttgccagcac gaangtgggc actctagagg   1140
gactgccggc gacaagtcgg aggaaggtgg ggatgacgtc aaatcatcat gccccttatg   1200
acctgggcta cacacgtgct acaatgggcg gtacaaaggg ctgcgaaccc gcgaggggga   1260
gcgaatccca aaaagccgct ctcagttcgg attgcaggct gcaactcgcc tgcatgaagc   1320
cggaatcgct agtaatcgcg gatcagcatg ccgcggtgaa tacgttcccg ggccttgtac   1380
acaccgcccg tcacaccacg agagcttgca acacccgaag tcggtgnggt aacccttacg   1440
ggagccagcc gccgaaggtg gggcaagtga ttggggtgaa gtcgtaacag ggtagcca     1498
```

<210> 5
<211> 20
<212> DNA
<213> Artificial Sequence
<220>
<223> Synthetic oligonucleotide 27f

<400> 5
agagtttgat cctgcctcag 20

<210> 6
<211> 19
<212> DNA
<213> Artificial Sequence
<220>
<223> Synthetic oligonucleotide 1492r

<400> 6
ggctaccttg ttacgactt 19

SEQUENCE LISTING

<110> SHINKO PANTEC CO.,LTD
<120> NOVEL MICROORGANISM AND PROCESS FOR TREATMENT OF ORGANIC SOLID MATTER USING THE MICROORGANISM
<130> Fuj-22103 EP
<140> EP03818540.1
<141> 2003-08-29
<150> JP2003209106
<151> 2003-08-27
<160> 6

<210> 1
<211> 1495
<212> DNA
<213> unknown
<220>
<223> 16SrRNA gene of Geobacillus sp.SPT4(FERM BP-08452)

<400> 1

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctgggggact tgagtgcaga agaggagagc ggaattccac     660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccct tcggggggac     1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg ggtgaagtc gtaacagggt agcca          1495
```

<210> 2
<211> 1498
<212> DNA
<213> Unknown
<220>
<223> 16SrRNA gene of Geobacillus sp.SPT5(FERM BP-08453)

<400> 2

```
agaacgctgg cggcgtgcct aatacatgca agtcgagcgg actgaatggg agcttgctct      60
tgttcggtca gcggcggacg ggtgagtaac acgtgggcaa cctgcccgca agaccgggat     120
aactccggga aaccggagct aataccggat aacaccgaag accgcatggt ctttggttga     180
aaggcggcgc aagctgccac ttgcggatgg gcccgcggcg cattagctag ttggtgaggt     240
aacggctcac caaggcgacg atgcgtagcc ggcctgagag ggtgaccggc cacactggga     300
ctgagacacg gcccagactc ctacgggagg cagcagtagg gaatcttccg caatgggcga     360
aagcctgacg gagcgacgcc gcgtgagcga agaaggcctt cgggtcgtaa agctctgttg     420
tgagggacga aggagcgccg tttgaagaag gcggcgcggt gacggtacct cacgaggaag     480
ccccggctaa ctacgtgcca gcagccgcgg taatacgtag ggcgagcgt tgtccggaat      540
tattgggcgt aaagcgcgcg caggcggttc cttaagtctg atgtgaaagc ccacggctca     600
accgtggagg gtcattggaa actgggggac ttgagtgcag gagaggagag cggaattcca     660
cgtgtagcgg tgaaatgcgt agagatgtgg aggaacacca gtggcgaagg cggctctctg     720
gcctgcaact gacgctgagg cgcgaaagcg tggggagcaa acaggattag ataccctggt     780
agtccacgcc gtaaacgatg agtgctaagt gttagagggg tcacaccctt tagtgctgca     840
gctaacgcga taagcactcc gcctggggag tacggccgca aggctgaaac tcaaaggaat     900
tgacgggggc ccgcacaagc ggtggagcat gtggtttaat tcgaagcaac gcgaagaacc     960
ttaccaggtc ttgacatccc ctgacaaccc aagagattgg gcgttccccc ttcgggggga    1020

cagggtgaca ggtggtgcat ggttgtcgtc agctcgtgtc gtgagatgtt gggttaagtc    1080
ccgcaacgag cgcaaccctt cgcctctagt tgccagcatt cggttgggca ctctagaggg    1140
actgccggcg acaagtcgga ggaaggtggg gatgacgtca aatcatcatg ccccttatga    1200
cctgggctac acacgtgcta caatgggcgg tacaaagggc tgcgaacccg cgagggggag    1260
cgaatcccaa aaagccgctc tcagttcgga ttgcaggctg caactcgcct gcatgaagcc    1320
ggaatcgcta gtaatcgcgg atcagcatgc cgcggtgaat acgttcccgg gccttgtaca    1380
caccgcccgt cacaccacga gagcttgcaa cacccgaagt cggtgaggca acccgtttcg    1440
ggagccagcc gccgaaggtg gggcaagtga ttggggtgaa gtcgtaacag ggtagcca      1498
```

<210> 3
<211> 1495

<212> DNA
<213> unknown
<220>
<223> 16SrRNA gene of Geobacillus sp.SPT6(FERM BP-08454)

<400> 3

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggggact tgagtgcaga agaggagagc ggaattccac     660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccccct tcgggggac    1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg gggtgaagtc gtaacagggt agcca         1495
```

<210> 4
<211> 1498
<212> DNA
<213> unknown
<220>
<223> 16SrRNA gene of Geobacillus sp.SPT7(FERM BP-08455)

<400> 4

```
gagaacgctg gcggcgtgcc taatacatgc aagtcgagcg gaccaaatcg gagcttgctc      60
tgatttggtc agcggcggac gggtgagtaa cacgtgggca acctgcccgc aagaccggga     120
taactccggg aaaccggagc taataccgga taacaccgaa gaccgcatgg tctttggttg     180
aaaggcggcc tttggctgtc acttgcggat gggcccgcgg cgcattagct agttggtgag     240
gtaacggctc accaaggcga cgatgcgtag ccggcctgag agggtgaccg gccacactgg     300
gactgagaca cggcccagac tcctacggga ggcagcagta gggaatcttc cgcaatgggc     360
gaaagcctga cggagcgacg ccgcgtgagc gaagaaggcc ttcgggtcgt aaagctctgt     420
tgtgagggac gaaggagcgc cgttcgaaga gggcggcgcg gtgacggtac ctcacgagga     480
agccccggct aactacgtgc cagcagccgc ggtaatacgt aggggcgagc gttgtccgga     540
attattgggc gtaaagcgcg cgcaggcggt tccttaagtc tgatgtgaaa gcccacggct     600
caaccgtgga gggtcattgg aaactggggg acttgagtgc aggagaggag agcggaattc     660
cacgtgtagc ggtgaaatgc gtagagatgt ggaggaacac cagtggcgaa ggcggctctc     720
tggcctgcaa ctgacgctga ggcgcgaaag cntggggagc aaacaggatt agataccctg     780
gtagtccacg ccgtaaacga tgagtgctaa gtgttagagg ggtcacaccc tttagtgctg     840
cagctaacgc gataagcact ccgcctgggg agtacggccg caaggctgaa actcaaagga     900
attgacgggg gcccgcacaa gcggtggagc atgtggttta attcgaagca acgcgaagaa     960
ccttaccagg tcttgacatc ccctgacaac ccaagagatt gggcgttccc ccttcggggg    1020
gacagggtga caggtggtgc atggttgtcg tcagctcgtg tcgtgagatg ttgggttaag    1080
```

```
tcccgcaacg agcgcaaccc tcgcctctag ttgccagcac gaangtgggc actctagagg     1140
gactgccggc gacaagtcgg aggaaggtgg ggatgacgtc aaatcatcat gccccttatg     1200
acctgggcta cacacgtgct acaatgggcg gtacaaaggg ctgcgaaccc gcgaggggga     1260
gcgaatccca aaaagccgct ctcagttcgg attgcaggct gcaactcgcc tgcatgaagc     1320
cggaatcgct agtaatcgcg gatcagcatg ccgcggtgaa tacgttcccg ggccttgtac     1380
acaccgcccg tcacaccacg agagcttgca acacccgaag tcggtgnggt aacccttacg     1440
ggagccagcc gccgaaggtg gggcaagtga ttggggtgaa gtcgtaacag ggtagcca       1498
```

<210> 5
<211> 20
<212> DNA
<213> Artificial Sequence
<220>
<223> Synthetic oligonucleotide 27f
<400> 5
agagtttgat cctgcctcag 20

<210> 6
<211> 19
<212> DNA
<213> Artificial Sequence
<220>
<223> Synthetic oligonucleotide 1492r

<400> 6
ggctaccttg ttacgactt 19

SEQUENCE LISTING

<110> SHINKO PANTEC CO., LTD
<120> NOVEL MICROORGANISM AND PROCESS FOR TREATMENT OF ORGANIC SOLID MATTER USING THE MICROORGANISM
<130> Fuj-22103 EP
<140> EP03818540.1
<141> 2003-08-29
<150> JP2003209106
<151> 2003-08-27
<160> 6

<210> 1
<211> 1495
<212> DNA
<213> Unknown
<220>
<223> 16SrRNA gene of Geobacillus sp.SPT4(FERM BP-08452)

<400> 1

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggggact tgagtgcaga agaggagagc ggaattccac     660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccct tcgggggac     1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg ggtgaagtc gtaacagggt agcca          1495
```

<210> 2

<211> 1498

<212> DNA

<213> Unknown

<220>

<223> 16SrRNA gene of Geobacillus sp.SPT5(FERM BP-08453)

<400> 2

```
agaacgctgg cggcgtgcct aatacatgca agtcgagcgg actgaatggg agcttgctct      60
tgttcggtca gcggcggacg ggtgagtaac acgtgggcaa cctgcccgca agaccgggat     120
aactccggga aaccggagct aataccggat aacaccgaag accgcatggt ctttggttga     180
aaggcggcgc aagctgccac ttgcggatgg gcccgcggcg cattagctag ttggtgaggt     240
aacggctcac caaggcgacg atgcgtagcc ggcctgagag ggtgaccggc cacactggga     300
ctgagacacg gcccagactc ctacgggagg cagcagtagg gaatcttccg caatgggcga     360
aagcctgacg gagcgacgcc gcgtgagcga agaaggcctt cgggtcgtaa agctctgttg     420
tgagggacga aggagcgccg tttgaagaag gcggcgcggt gacggtacct cacgaggaag     480
ccccggctaa ctacgtgcca gcagccgcgg taatacgtag ggcgagcgt tgtccggaat     540
tattgggcgt aaagcgcgcg caggcggttc cttaagtctg atgtgaaagc ccacggctca     600
accgtggagg gtcattggaa actgggggac ttgagtgcag gagaggagag cggaattcca     660
cgtgtagcgg tgaaatgcgt agagatgtgg aggaacacca gtggcgaagg cggctctctg     720
gcctgcaact gacgctgagg cgcgaaagcg tggggagcaa acaggattag ataccctggt     780
agtccacgcc gtaaacgatg agtgctaagt gttagagggg tcacaccctt tagtgctgca     840
gctaacgcga taagcactcc gcctggggag tacggccgca aggctgaaac tcaaaggaat     900
tgacgggggc ccgcacaagc ggtggagcat gtggtttaat tcgaagcaac gcgaagaacc     960
ttaccaggtc ttgacatccc ctgacaaccc aagagattgg gcgttccccc ttcgggggga    1020
```

```
cagggtgaca ggtggtgcat ggttgtcgtc agctcgtgtc gtgagatgtt gggttaagtc    1080
ccgcaacgag cgcaaccctt cgcctctagt tgccagcatt cggttgggca ctctagaggg    1140
actgccggcg acaagtcgga ggaaggtggg gatgacgtca aatcatcatg ccccttatga    1200
cctgggctac acacgtgcta caatgggcgg tacaaagggc tgcgaacccg cgagggggag    1260
cgaatcccaa aaagccgctc tcagttcgga ttgcaggctg caactcgcct gcatgaagcc    1320
ggaatcgcta gtaatcgcgg atcagcatgc cgcggtgaat acgttcccgg gccttgtaca    1380
caccgcccgt cacaccacga gagcttgcaa cacccgaagt cggtgaggca acccgtttcg    1440
ggagccagcc gccgaaggtg gggcaagtga ttggggtgaa gtcgtaacag ggtagcca      1498
```

<210> 3

<211> 1495

<212> DNA

<213> unknown

<220>

<223> 16srRNA gene of Geobacillus sp.SPT6(FERM BP-08454)

<400> 3

```
gaacgctggc ggcgtgccta atacatgcaa gtcgagcgga ccggacagga gcttgctctt      60
gttcggttag cggcggacgg gtgagtaaca cgtgggcaac ctacccgtaa gaccgggata     120
actccgggaa accggagcta ataccggata acaccgaaga ccgcatggtc ttcggttgaa     180
aggcggcttt ggctgtcact tacggatggg cccgcggcgc attagctagt tggtgaggta     240
acggctcacc aaggcgacga tgcgtagccg gcctgagagg gtgaccggcc acactgggac     300
tgagacacgg cccagactcc tacgggaggc agcagtaggg aatcttccgc aatggacgaa     360
agtctgacgg agcgacgccg cgtgagcgaa gaaggtcttc ggatcgtaaa gctctgttgt     420
tagggaagaa gaagtaccgt tcgaataggg cggtacggtg acggtaccta acgagaaagc     480
cccggctaac tacgtgccag cagccgcggt aatacgtagg ggcgagcgtt gtccggaatt     540
attgggcgta aagcgcgcgc aggcggtccc ttaagtctga tgtgaaagcc cacggctcaa     600
ccgtggaggg tcattggaaa ctggggactt gagtgcagaa gaggagagcg gaattccacg     660
gtgtagcggt gaaatgcgta gagatgtgga ggaacaccag tggcgaaggc ggctctctgg     720
tctgtaactg acgctgaggc gcgaaagcgt ggggagcaaa caggattaga taccctggta     780
gtccacgccg taaacgatga gtgctaagtg ttagaggggt caaacccttt agtgctgcag     840
ctaacgcgtt aagcactccg cctggggagt acggccgcaa ggctgaaact caaaggaatt     900
gacgggggcc cgcacaagcg gtggagcatg tggtttaatt cgaagcaacg cgaagaacct     960
taccaggtct tgacatcccc tgacaaccct agagataggg cgttcccct tcgggggac     1020
agggtgacag gtggtgcatg gttgtcgtca gctcgtgtcg tgagatgttg ggttaagtcc    1080
cgcaacgagc gcaaccctcg accttagttg ccagcattca gttgggcact ctaaggtgac    1140
tgccgatgac aaatcggagg aaggtgggga tgacgtcaaa tcatcatgcc ccttatgacc    1200
tgggctacac acgtgctaca atgggcggta caaagggctg cgaacccgcg agggggagcg    1260
aatcccaaaa agccgctctc agttcggatt gcaggctgca actcgcctgc atgaagccgg    1320
aatcgctagt aatcgcggat cagcatgccg cggtgaatac gttcccgggc cttgtacaca    1380
ccgcccgtca caccacgaga gcttgcaaca cccgaagtcg gtgaggtaac cctttcggga    1440
gccagccgcc gaaggtgggg caagtgattg gggtgaagtc gtaacagggt agcca         1495
```

<210> 4
<211> 1498
<212> DNA
<213> unknown
<220>
<223> 16SrRNA gene of Geobacillus sp.SPT7(FERM BP-08455)

<400> 4

```
gagaacgctg gcggcgtgcc taatacatgc aagtcgagcg gaccaaatcg gagcttgctc      60
tgatttggtc agcggcggac gggtgagtaa cacgtgggca acctgcccgc aagaccggga     120
taactccggg aaaccggagc taataccgga taacaccgaa gaccgcatgg tctttggttg     180
aaaggcggcc tttggctgtc acttgcggat gggcccgcgg cgcattagct agttggtgag     240
gtaacggctc accaaggcga cgatgcgtag ccggcctgag agggtgaccg gccacactgg     300
gactgagaca cggcccagac tcctacggga ggcagcagta gggaatcttc cgcaatgggc     360
gaaagcctga cggagcgacg ccgcgtgagc gaagaaggcc ttcgggtcgt aaagctctgt     420
tgtgagggac gaaggagcgc cgttcgaaga gggcggcgcg gtgacggtac ctcacgagga     480
agccccggct aactacgtgc cagcagccgc ggtaatacgt aggggcgagc gttgtccgga     540
attattgggc gtaaagcgcg cgcaggcggt tccttaagtc tgatgtgaaa gcccacggct     600
caaccgtgga gggtcattgg aaactggggg acttgagtgc aggagaggag agcggaattc     660
cacgtgtagc ggtgaaatgc gtagagatgt ggaggaacac cagtggcgaa ggcggctctc     720
tggcctgcaa ctgacgctga ggcgcgaaag cntggggagc aaacaggatt agataccctg     780
gtagtccacg ccgtaaacga tgagtgctaa gtgttagagg ggtcacaccc tttagtgctg     840
cagctaacgc gataagcact ccgcctgggg agtacggccg caaggctgaa actcaaagga     900
attgacgggg gcccgcacaa gcggtggagc atgtggttta attcgaagca acgcgaagaa     960
ccttaccagg tcttgacatc ccctgacaac ccaagagatt gggcgttccc ccttcggggg    1020
gacagggtga caggtggtgc atggttgtcg tcagctcgtg tcgtgagatg ttgggttaag    1080

tcccgcaacg agcgcaaccc tcgcctctag ttgccagcac gaangtgggc actctagagg    1140
gactgccggc gacaagtcgg aggaaggtgg ggatgacgtc aaatcatcat gccccttatg    1200
acctgggcta cacacgtgct acaatgggcg gtacaaaggg ctgcgaaccc gcgaggggga    1260
gcgaatccca aaaagccgct ctcagttcgg attgcaggct gcaactcgcc tgcatgaagc    1320
cggaatcgct agtaatcgcg gatcagcatg ccgcggtgaa tacgttcccg ggccttgtac    1380
acaccgcccg tcacaccacg agagcttgca acacccgaag tcggtgnggt aacccttacg    1440
ggagccagcc gccgaaggtg gggcaagtga ttggggtgaa gtcgtaacag ggtagcca      1498
```

<210> 5
<211> 20
<212> DNA
<213> Artificial Sequence
<220>
<223> synthetic oligonucleotide 27f

<400> 5
agagtttgat cctgcctcag 20

<210> 6
<211> 19
<212> DNA
<213> Artificial Sequence

<220>
<223> Synthetic oligonucleotide 1492r

<400> 6
ggctaccttg ttacgactt 19

SEQUENCE LISTING

<110> SHINKO PANTEC CO. , LTD
<120> NOVEL MICROORGANISM AND PROCESS FOR TREATMENT OF ORGANIC SOLID MATTER USING THE MICROORGANISM
<130> F-1910
<160> 6

<210> 1
<211> 1495
<212> DNA
<213> Geobacillus sp.SPT4

<400> 1

```
gaacgctggc ggcgtgccta atacatgcaa gtcgagcgga ccggacagga gcttgctctt 60
gttcggttag cggcggacgg gtgagtaaca cgtgggcaac ctacccgtaa gaccgggata 120
actccgggaa accggagcta ataccggata acaccgaaga ccgcatggtc ttcggttgaa 180
aggcggcttt ggctgtcact tacggatggg cccgcggcgc attagctagt tggtgaggta 240
acggctcacc aaggcgacga tgcgtagccg gcctgagagg gtgaccggcc acactgggac 300
tgagacacgg cccagactcc tacgggaggc agcagtaggg aatcttccgc aatggacgaa 360
agtctgacgg agcgacgccg cgtgagcgaa gaaggtcttc ggatcgtaaa gctctgttgt 420
tagggaagaa gaagtaccgt tcgaataggg cggtacggtg acggtaccta acgagaaagc 480
cccggctaac tacgtgccag cagccgcggt aatacgtagg ggcgagcgtt gtccggaatt 540
attgggcgta aagcgcgcgc aggcggtccc ttaagtctga tgtgaaagcc cacggctcaa 600
ccgtggaggg tcattggaaa ctgggggact tgagtgcaga agaggagagc ggaattccac 660
gtgtagcggt gaaatgcgta gagatgtgga ggaacaccag tggcgaaggc ggctctctgg 720
tctgtaactg acgctgaggc gcgaaagcgt ggggagcaaa caggattaga taccctggta 780
gtccacgccg taaacgatga gtgctaagtg ttagaggggt caaacccttt agtgctgcag 840
ctaacgcgtt aagcactccg cctggggagt acggccgcaa ggctgaaact caaaggaatt 900
gacgggggcc cgcacaagcg gtggagcatg tggtttaatt cgaagcaacg cgaagaacct 960
taccaggtct tgacatcccc tgacaaccct agagataggg cgttcccct tcgggggac 1020
agggtgacag gtggtgcatg gttgtcgtca gctcgtgtcg tgagatgttg ggttaagtcc 1080
cgcaacgagc gcaaccctcg accttagttg ccagcattca gttgggcact ctaaggtgac 1140
tgccgatgac aaatcggagg aaggtgggga tgacgtcaaa tcatcatgcc ccttatgacc 1200
tgggctacac acgtgctaca atgggcggta caaagggctg cgaacccgcg agggggagcg 1260
aatcccaaaa agccgctctc agttcggatt gcaggctgca actcgcctgc atgaagccgg 1320
aatcgctagt aatcgcggat cagcatgccg cggtgaatac gttcccgggc cttgtacaca 1380
ccgcccgtca caccacgaga gcttgcaaca cccgaagtcg gtgaggtaac cctttcggga 1440
gccagccgcc gaaggtgggg caagtgattg ggtgaagtc gtaacagggt agcca 1495
```

<210> 2
<211> 1498
<212> DNA
<213> Geobacillus sp. SPT5

<400> 2

agaacgctgg cggcgtgcct aatacatgca agtcgagcgg actgaatggg agcttgctct 60
tgttcggtca gcggcggacg ggtgagtaac acgtgggcaa cctgcccgca agaccgggat 120
aactccggga aaccggagct aataccggat aacaccgaag accgcatggt ctttggttga 180
aaggcggcgc aagctgccac ttgcggatgg gcccgcggcg cattagctag ttggtgaggt 240
aacggctcac caaggcgacg atgcgtagcc ggcctgagag ggtgaccggc cacactggga 300
ctgagacacg gcccagactc ctacgggagg cagcagtagg gaatcttccg caatgggcga 360
aagcctgacg gagcgacgcc gcgtgagcga agaaggcctt cgggtcgtaa agctctgttg 420
tgagggacga aggagcgccg tttgaagaag gcggcgcggt gacggtacct cacgaggaag 480
ccccggctaa ctacgtgcca gcagccgcgg taatacgtag ggcgagcgt tgtccggaat 540
tattgggcgt aaagcgcgcg caggcggttc cttaagtctg atgtgaaagc ccacggctca 600
accgtggagg gtcattggaa actgggggac ttgagtgcag gagaggagag cggaattcca 660
cgtgtagcgg tgaaatgcgt agagatgtgg aggaacacca gtggcgaagg cggctctctg 720
gcctgcaact gacgctgagg cgcgaaagcg tggggagcaa acaggattag ataccctggt 780
agtccacgcc gtaaacgatg agtgctaagt gttagagggg tcacaccctt tagtgctgca 840
gctaacgcga taagcactcc gcctggggag tacggccgca aggctgaaac tcaaaggaat 900
tgacgggggc ccgcacaagc ggtggagcat gtggtttaat tcgaagcaac gcgaagaacc 960
ttaccaggtc ttgacatccc ctgacaaccc aagagattgg gcgttccccc ttcgggggga 1020
cagggtgaca ggtggtgcat ggttgtcgtc agctcgtgtc gtgagatgtt gggttaagtc 1080
ccgcaacgag cgcaaccctt cgcctctagt tgccagcatt cggttgggca ctctagaggg 1140
actgccggcg acaagtcgga ggaaggtggg gatgacgtca aatcatcatg ccccttatga 1200
cctgggctac acacgtgcta caatgggcgg tacaaagggc tgcgaacccg cgagggggag 1260
cgaatcccaa aaagccgctc tcagttcgga ttgcaggctg caactcgcct gcatgaagcc 1320
ggaatcgcta gtaatcgcgg atcagcatgc cgcggtgaat acgttcccgg gccttgtaca 1380
caccgcccgt cacaccacga gagcttgcaa cacccgaagt cggtgaggca acccgtttcg 1440
ggagccagcc gccgaaggtg ggcaagtga ttggggtgaa gtcgtaacag ggtagcca 1498

<210> 3
<211> 1495
<212> DNA
<213> Geobacillus sp.SPT6

<400> 3

gaacgctggc ggcgtgccta atacatgcaa gtcgagcgga ccggacagga gcttgctctt 60
gttcggttag cggcggacgg gtgagtaaca cgtgggcaac ctacccgtaa gaccgggata 120
actccgggaa accggagcta ataccggata acaccgaaga ccgcatggtc ttcggttgaa 180
aggcggcttt ggctgtcact tacggatggg cccgcggcgc attagctagt tggtgaggta 240
acggctcacc aaggcgacga tgcgtagccg gcctgagagg gtgaccggcc acactgggac 300
tgagacacgg cccagactcc tacgggaggc agcagtaggg aatcttccgc aatggacgaa 360
agtctgacgg agcgacgccg cgtgagcgaa gaaggtcttc ggatcgtaaa gctctgttgt 420
tagggaagaa gaagtaccgt tcgaataggg cggtacggtg acggtaccta acgagaaagc 480
cccggctaac tacgtgccag cagccgcggt aatacgtagg ggcgagcgtt gtccggaatt 540
attgggcgta aagcgcgcgc aggcggtccc ttaagtctga tgtgaaagcc cacggctcaa 600
ccgtggaggg tcattggaaa ctgggggact tgagtgcaga agaggagagc ggaattccac 660
gtgtagcggt gaaatgcgta gagatgtgga ggaacaccag tggcgaaggc ggctctctgg 720
tctgtaactg acgctgaggc gcgaaagcgt ggggagcaaa caggattaga taccctggta 780
gtccacgccg taaacgatga gtgctaagtg ttagaggggt caaacccttt agtgctgcag 840
ctaacgcgtt aagcactccg cctggggagt acggccgcaa ggctgaaact caaaggaatt 900
gacgggggcc cgcacaagcg gtggagcatg tggtttaatt cgaagcaacg cgaagaacct 960
taccaggtct tgacatcccc tgacaaccct agagataggg cgttccccct tcgggggac 1020
agggtgacag gtggtgcatg gttgtcgtca gctcgtgtcg tgagatgttg ggttaagtcc 1080
cgcaacgagc gcaaccctcg accttagttg ccagcattca gttgggcact ctaaggtgac 1140
tgccgatgac aaatcggagg aaggtgggga tgacgtcaaa tcatcatgcc ccttatgacc 1200
tgggctacac acgtgctaca atgggcggta caaagggctg cgaacccgcg agggggagcg 1260
aatcccaaaa agccgctctc agttcggatt gcaggctgca actcgcctgc atgaagccgg 1320
aatcgctagt aatcgcggat cagcatgccg cggtgaatac gttcccgggc cttgtacaca 1380
ccgcccgtca caccacgaga gcttgcaaca cccgaagtcg gtgaggtaac cctttcggga 1440
gccagccgcc gaaggtgggg caagtgattg ggtgaagtc gtaacagggt agcca 1495

<210> 4
<211> 1498
<212> DNA
<213> Geobacillus sp.SPT7

<400> 4

```
gagaacgctg gcggcgtgcc taatacatgc aagtcgagcg gaccaaatcg gagcttgctc   60
tgatttggtc agcggcggac gggtgagtaa cacgtgggca acctgcccgc aagaccggga  120
taactccggg aaaccggagc taataccgga taacaccgaa gaccgcatgg tctttggttg  180
aaaggcggcc tttggctgtc acttgcggat gggcccgcgg cgcattagct agttggtgag  240
gtaacggctc accaaggcga cgatgcgtag ccggcctgag agggtgaccg gccacactgg  300
gactgagaca cggcccagac tcctacggga ggcagcagta gggaatcttc cgcaatgggc  360
gaaagcctga cggagcgacg ccgcgtgagc gaagaaggcc ttcgggtcgt aaagctctgt  420
tgtgagggac gaaggagcgc cgttcgaaga gggcggcgcg gtgacggtac ctcacgagga  480
agccccggct aactacgtgc cagcagccgc ggtaatacgt aggggcgagc gttgtccgga  540
attattgggc gtaaagcgcg cgcaggcggt tccttaagtc tgatgtgaaa gcccacggct  600
caaccgtgga gggtcattgg aaactgggg acttgagtgc aggagaggag agcggaattc   660
cacgtgtagc ggtgaaatgc gtagagatgt ggaggaacac cagtggcgaa ggcggctctc  720
tggcctgcaa ctgacgctga ggcgcgaaag cntggggagc aaacaggatt agataccctg  780
gtagtccacg ccgtaaacga tgagtgctaa gtgttagagg ggtcacaccc tttagtgctg  840
cagctaacgc gataagcact ccgcctgggg agtacggccg caaggctgaa actcaaagga  900
attgacgggg gcccgcacaa gcggtggagc atgtggttta attcgaagca acgcgaagaa  960
ccttaccagg tcttgacatc ccctgacaac ccaagagatt gggcgttccc ccttcggggg 1020
gacagggtga caggtggtgc atggttgtcg tcagctcgtg tcgtgagatg ttgggttaag 1080
tcccgcaacg agcgcaaccc tcgcctctag ttgccagcac gaangtgggc actctagagg 1140
gactgccggc gacaagtcgg aggaaggtgg ggatgacgtc aaatcatcat gccccttatg 1200
acctgggcta cacacgtgct acaatgggcg gtacaaaggg ctgcgaaccc gcgaggggga 1260
gcgaatccca aaaagccgct ctcagttcgg attgcaggct gcaactcgcc tgcatgaagc 1320
cggaatcgct agtaatcgcg gatcagcatg ccgcggtgaa tacgttcccg ggccttgtac 1380
acaccgcccg tcacaccacg agagcttgca acacccgaag tcggtgnggt aacccttacg 1440
ggagccagcc gccgaaggtg ggcaagtga ttggggtgaa gtcgtaacag ggtagcca    1498
```

<210> 5
<211> 20
<212> DNA
<213> Artificial Sequence

<400> 5
agagtttgat cctgcctcag 20

<210> 6
<211> 19
<212> DNA
<213> Artificial Sequence

<400> 6
ggctaccttg ttacgactt 19

## Description

### Technical Field

The present invention relates to a novel microorganism having an ability to produce a solubilizing enzyme which solubilizes a variety of organic solid matter such as biological sludge including raw sludge and excess sludge discharged from sewage treating processes at sewage treatment plants, night soil treatment plants or the like, or organic sludge discharged from production processes or wastewater treatment processes at food plants, chemical plants or the like; and a process for treatment of organic solid matter using the microorganism.

### Background Art

Conventionally, there have been hitherto reports on the processes for treating a variety of organic solid matter such as biological sludge including raw sludge and excess sludge discharged from sewage treating processes at sewage treatment plants, night soil treatment plants or the like, or organic sludge discharged from production processes or drainage treatment processes at food plants, chemical plants or the like, which are the processes for solubilization of organic solid matter, wherein organic solid matter is biologically decomposed by the action of bacteria or the like; and the strains which are expected to be applied thereto, as described in the following Prior Art Documents 1 to 4.
Prior Art Document 1: JP-A No. 7-184640
Prior Art Document 2: Report on Development of New Drainage Treatment System Utilizing Biotechnology (Part on Sewage), pp. 73-77, Public Works Research Center (Japan) (Feb. 1991).
Prior Art Document 3: Shigeru Kume and Yusaku Fujio, "Digestion of Municipal Sewage Sludge by a Mixture of Thermophilic Bacilli and Their Culture Extract," J. Gen. Appl. Microbiol., 36, 189-194 (1990).
Prior Art Document 4: Yusaku Fujio and Shigeru Kume, "Isolation and Identification of Thermophilic Bacteria from Sewage Sludge Compost," Journal of Fermentation and Bioengineering, Vol. 72, No. 5, 334-337 (1991).
The above-mentioned Prior Art Document 1 relates to a process for treatment of a yeast extract residue using a strain producing an enzyme which specifically decomposes the yeast extract residue, namely, a strain belonging to genus Oerskovia sp. 24 (FERM P-13692).
The above-mentioned Prior Art Document 2 discloses an anaerobic strain belonging to Clostridium bifermentans, which achieves specific solubilization of sterilized excess sludge under anaerobic conditions.
Further, the above-mentioned Prior Art Documents 3 and 4 disclose digestion of sludge using a strain belonging to Bacillus stearothermophilus which is isolated from sewage sludge compost aerobically under high temperature conditions.
However, according to Prior Art Document 1, there is a problem that the subject that can be treated by decomposition is substantially limited to the yeast extract residue.
Also, the strains disclosed in Prior Art Documents 2 to 4 are known to show low treatment efficiencies because they respectively show efficiencies for sludge solubilization of 25% after 10 days and of about 40 to 50% after 20 days, thus requiring a long period of time to achieve a predetermined solubilization rate. Further, such low treatment efficiency still remains as a problem to be solved in the industrial use of the strains.

### Disclosure of the Invention

In order to solve these problems, the present inventors have searched for a microorganism which has an excellent effect for solubilizing organic solid matter such as sludge and which can increase the treatment efficiency by significantly reducing the treatment time to achieve a predetermined solubilization rate.
As a result, they isolated a novel microorganism belonging to genus Geobacillus which satisfies such requirements, thus completing the present invention.
Thus, the invention described in claim 1 is a novel microorganism belonging to genus Geobacillus, which is any one of Geobacillus sp. SPT4 (FERM BP-08452), Geobacillus sp. SPT5 (FERM BP-08453), Geobacillus sp. SPT6 (FERM BP-08454) and Geobacillus sp. SPT7 (FERM BP-08455) having an ability to produce a solubilizing enzyme for solubilizing organic solid matter such as organic sludge or biological sludge.
The novel microorganism belonging to genus Geobacillus, which has an ability to produce a solubilizing enzyme for solubilizing organic solid matter such as organic sludge or biological sludge has the following mycological characteristics:

A. Morphological characteristics

(1) Shape and size of cell: rod-shaped cell with a width of 0.7 to 0.8 μm and a length of 2.0 to 4.0 μm

(2) Presence or absence of motility: present
(3) Presence or absence of spore: present

B. Cultivating characteristics (Nutrient agar plate culture)

(1) Colony morphology: circular, entirely smooth edge, low convex
(2) Color: cream color
(3) Gloss: present

C. Physiological characteristics

(1) Gram staining property: +
(2) Nitrate reduction: -
(3) Indole production: -
(4) Hydrogen sulfide production: -
(5) Use of citric acid: -
(6) Urease: -
(7) Oxidase: +
(8) Catalase: +
(9) Attitude to oxygen: aerobic
(10) O□F test (glucose): -/-
(11) Production of acid and gas from saccharides D-glucose: acid (+)/gas (-).

A novel microorganism belonging to genus Geobacillus, which has an ability to produce a solubilizing enzyme for solubilizing organic solid matter such as organic sludge or biological sludge has the following mycological characteristics:

A. Morphological characteristics:

(1) Shape and size of cell: rod-shaped cell with a width of 0.7 to 0.8 μm and a length of 2.0 to 4.0 μm
(2) Presence or absence of motility: present
(3) Presence or absence of spore: present

B. Cultivating characteristics (Nutrient agar plate culture)

(1) Colony morphology: circular, entirely smooth edge, low convex
(2) Color: cream color
(3) Gloss: present

C. Physiological characteristics

(1) Gram staining property: +
(2) Nitrate reduction: -
(3) Indole production: -
(4) Hydrogen sulfide production: -
(5) Use of citric acid: -
(6) Urease: -
(7) Oxidase: +
(8) Catalase: +
(9) Attitude to oxygen: aerobic
(10) O-F test (glucose): -/-
(11) Production of acid and gas from saccharides D-glucose: acid (+)/gas (-)
(12) Fermentability test

(a) D-glucose: +
(b) D-fructose: +
(c) D-mannose: +
(d) D-sorbitol: -

(e) Inositol: -
(f) Maltose: +
(g) Trehalose: +

(13) Other physiological characteristics

(a) β-Galactosidase activity: -
(b) Arginine dihydrolase activity: -
(c) Lysine dicarboxylase activity: -
(d) Tryptophan deaminase activity: -
(e) Acetoin production: -
(f) Gelatinase activity: +
(g) Ornithine dicarboxylase activity: -.

A novel microorganism is any one of Geobacillus sp. SPT4 (FERM BP-08452); Geobacillus sp. SPT5 (FERM BP-08453); Geobacillus sp. SPT6 (FERM BP-08454); and Geobacillus sp. SPT7 (FERM BP-08455).

A a novel microorganism Geobacillus sp. SPT4 (FERM BP-08452) belonging to genus Geobacillus, which has an ability to produce a solubilizing enzyme for solubilizing organic solid matter such as organic sludge or biological sludge, has a base sequence of 16SrRNA gene as described in SEQ ID No. 1 in the sequence listing.

A novel microorganism Geobacillus sp. SPT5 (FERM BP-08453) belonging to genus Geobacillus, which has an ability to produce a solubilizing enzyme for solubilizing organic solid matter such as organic sludge or biological sludge, has a base sequence of 16SrRNA gene as described in SEQ ID No. 2 in the sequence listing.

A novel microorganism Geobacillus sp. SPT7 (FERM BP-08455) belonging to genus Geobacillus, which has an ability to produce a solubilizing enzyme for solubilizing organic solid matter such as organic sludge or biological sludge, has a base sequence of 16SrRNA gene as described in SEQ ID No. 4 in the sequence listing.

The invention also comprises a process for treatment of organic solid matter in which organic solid matter such as organic sludge or biological sludge is solubilized using a novel microorganism of at least one of Geobacillus sp. SPT4 (FERM BP-08452), Geobacillus sp. SPT5 (FERM BP-08453), Geobacillus sp. SPT6 (FERM BP-08454) and Geobacillus sp. SPT7 (FERM BP-08455).

Furthermore, the invention comprises a process for treatment of organic solid matter in which organic solid matter such as organic sludge or biological sludge is solubilized by a mixture of microorganisms comprising any one of the novel microorganisms Geobacillus sp. SPT4 (FERM BP-08452), Geobacillus sp. SPT5 (FERM BP-08453) or Geobacillus sp. SPT6 (FERM BP-08454) and the novel microorganism Geobacillus sp. SPT7 (FERM BP-08455).

According to the present invention, as described above, there are provided novel microorganisms belonging to genus Geobacillus such as Geobacillus sp.SPT4, Geobacillus sp.SPT5, Geobacillus sp. SPT6 and Geobacillus sp. SPT7, which have the property of solubilizing organic solid matter such as biological sludge or organic sludge.

In particular, since the present invention exhibits an excellent effect for sludge solubilization within an extremely short time period of about one day, in the case of applying the present invention to an actual apparatus, there is obtained an effect of shortening the time for starting up operation of the apparatus.

Further, since the present invention exhibits an excellent solubilizing effect at a mild temperature of about 50°C to 65°C, in the case of applying the present invention to an actual apparatus, there is obtained an effect of significantly improving economic efficiency in the aspect of energy.

Furthermore, it is possible to efficiently solubilize a variety of organic solid matter such as biological sludge including raw sludge and excess sludge that are discharged from sewage treatment processes at sewage treatment plants, night soil treatment plants or the like, or organic sludge that is discharged from production processes or from wastewater treatment processes at food plants, chemical plants or the like, by means of a process for treatment of solubilizing organic solid matter such as organic sludge or biological sludge using these novel microorganisms.

Especially, when a mixture of microorganisms comprising Geobacillus sp. SPT7 and any one of Geobacillus sp. SPT4, Geobacillus sp. SPT5 and Geobacillus sp. SPT6 is used, there is obtained an effect of further improving the rate of solubilization of sludge.

Brief Description of the Drawings

Fig. 1 is a schematic block diagram illustrating an apparatus for treatment of organic wastewater using a microorganism in an embodiment.

Fig. 2 is a schematic block diagram illustrating an apparatus for treatment of organic wastewater in another embodiment.

Fig. 3 is a schematic block diagram illustrating an apparatus for treatment of organic wastewater in another embodiment.

Fig. 4 is a schematic block diagram illustrating an apparatus for treatment of organic wastewater in another embodiment.

Fig. 5 is a schematic block diagram illustrating an apparatus for treatment of organic wastewater in another embodiment.

Fig. 6 is a schematic block diagram illustrating an apparatus for treatment of organic wastewater in another embodiment.

Fig. 7 is a schematic block diagram illustrating an apparatus for treatment of organic wastewater in another embodiment.

Fig. 8 is a block diagram of the batch type treatment process carried out in the apparatus for treatment of Fig. 7.

Fig. 9 is a schematic block diagram illustrating an apparatus for treatment of organic wastewater in another embodiment.

Fig. 10 is a schematic block diagram illustrating an apparatus for treatment of organic wastewater in another embodiment.

Fig. 11 is a schematic block diagram illustrating an apparatus for treatment of organic wastewater in another embodiment.

Best Mode for Carrying Out the Invention

Hereinafter, embodiments of the present invention will be described.

[Strain screening]

Isolation of microorganism in each of the below-described embodiments of the invention was carried out as follows.

From the excess sludge discharged from a sewage treatment plant, sludge for strain separation was collected. Thus collected sludge was diluted $1\times10^{-2}$ to $10^{-5}$ times with sterilized water and then applied on an agar plate containing a nutrient agar medium (manufactured by Oxoido Limited) . This plate was incubated overnight at 60°C to obtain single colony.

For the obtained single colony, the ability to decompose skim milk and the ability to decompose sterilized sludge were examined based on the presence or absence of halo formation in a substrate complex medium, and among the halo-forming strains, four strains showing particularly high ability of sludge decomposition were selected as characteristic strains. Specifically, determination was made with naked eyes in agar media mixed with 0.1 wt%/vol% of the strains, respectively, according to the extent of halo (plaque) formation.

Calibration for the ability to decompose skim milk was carried out by a modified method in R. Beaudet, C. Gagnon, J.G. Bisaillon and M. Ishaque, "Microbiological Aspects of Aerobic Thermophilic Treatment of Swine waste," Applied and Environmental Microbiology, pp.971-976 (April, 1990).

(Embodiment 1)

One strain among the selected four strains was tested for the mycological characteristics in the manner as described above. The mycological characteristics (morphological characteristics, cultivating characteristics and physiological characteristics) are as follows:

A. Morphological characteristics

(1) Shape and size of cell: rod-shaped cell with a width of 0.7 to 0.8 μm and a length of 2.0 to 4.0 μm
(2) Presence or absence of motility: present
(3) Presence or absence of spore: present

B. Cultivating characteristics (Nutrient agar plate culture)

(1) Colonymorphology: circular, entirely smooth edge, low convex
(2) Color: cream color
(3) Gloss: present

C. Physiological characteristics

(1) Gram staining property: +
(2) Nitrate reduction: -
(3) VP test: -
(4) Indole production: -
(5) Hydrogen sulfide production: -
(6) Hydrolysis of starch: -
(7) Use of citric acid: -
(8) Urease: -
(9) Oxidase: +
(10) Catalase: +
(11) Growth conditions

(a) pH: 6.0 to 8.0
(b) Temperature: 45°C to 65°C

(12) Attitude to oxygen: aerobic
(13) O-F test (Hugh Leifson method): - /-
(14) Production of acid and gas from saccharides D-glucose: acid (+)/gas (-)
(15) Fermentability test

(a) Glycerol: -
(b) L-arabinose: -
(c) D-xylose : -
(d) D-galactose: -
(e) D-glucose: +
(f) D-fructose: +
(g) D-mannose: +
(h) D-mannitol: +
(i) Inositol: -
(j) Sorbitol: -
(k) Maltose: +
(l) Lactose: +
(m) Sucrose: -
(n) Trehalose: +

(16) Other physiological characteristics

(a) β-Galactosidase activity: -
(b) Arginine dihydrolase activity: -
(c) Lysine dicarboxylase activity: -
(d) Tryptophan deaminase activity: -
(e) Acetoin production: -
(f) Gelatinase activity: +
(g) Ornithine dicarboxylase activity: - .

Further, the measurement of pH and temperature for growth of the strain among the above-described physiological characteristics was carried out as follows.

A nutrient agar medium (manufactured by Oxoido Limited) was prepared in which pH was adjusted to the pH under test (6.0, 6.5, 7.0, 7.5 and 8.0) using 0.1 N hydrochloric acid (manufactured by Wako Pure Chemical Industries, Ltd.) and 0.1 N sodium hydroxide (manufactured by Wako Pure Chemical Industries, Ltd.), or either of them. To this medium, the strain of the present embodiment was inoculated, the amount of cell growth for the strain cultivated at 60°C for 15 hours was observed, and the degree of growth was measured qualitatively. The results are presented in the following Table 1.

Table 1 +: positive ±: weakly positive

| pH | Degree of growth |
|---|---|
| 6.0 | + |
| 6.5 | + |
| 7.0 | + |
| 7.5 | + |
| 8.0 | ± |

As obvious from Table 1 above, it was found that the strain of the present embodiment grows at a pH in the range of 6.0 to 8.0.

Next, a nutrient agar medium (manufactured by Oxoido Limited) which was adjusted to pH 6.5 using 0.1 N hydrochloric acid (manufactured by Wako Pure Chemical Industries, Ltd.) and 0.1 N sodium hydroxide (manufactured by Wako Pure Chemical Industries, Ltd.), or either of them, was prepared. To this medium, the strain of the present embodiment was inoculated, and the amount of cell growth of the strain cultivated at the temperatures under test (37°C, 45°C, 50°C, 60 °C and 65 °C) for 15 hours was observed to measure the degree of growth qualitatively. The results are presented in the following Table 2.

Table 2 +: positive ±: weakly positive - : negative

| Temperature | Degree of growth |
|---|---|
| 37°C | - |
| 45°C | ± |
| 50°C | + |
| 60°C | + |
| 65°C | + |

As obvious from Table 2 above, it was found that the strain of the present embodiment grows at a temperature in the range of 50°C to 65°C.

Furthermore, the base sequence of 16SrRNA gene of this strain was interpreted. The sequence is as described in SEQ ID No. 1 in the sequence listing. In this interpretation, 16SrRNA gene of the strain of the present embodiment was first amplified by the PCR method. PCR was carried out with the DNA purified from the strain of the present embodiment which had been subjected to shaking incubation at 60 °C for 6 hours, according to the method by Heng Zhu, et al. (Heng Zhu, Feng Qu and Li-Husang Zhu, "Isolation of genomic DNAs from 1993), as a template. For the primer, the two synthetic oligonucleotides (27f, 1492r) described in "Classification of Microorganisms and Experimentation for Classification: Based on Techniques from Molecular Genetics and Molecular Biology" (edited by Kenichiro Suzuki, Akira Hiraishi and Akira Yokota, Springer-Verlag, Tokyo) were used. The base sequences of these primers are as described in SEQ ID Nos. 5 and 6 in the sequence listing.

The PCR reaction was carried out as follows. A reaction solution comprising 1×PCR Buffer (Toyobo Co., Ltd.), 0.2 mM dNTPs (Toyobo Co., Ltd.), 1 mM $MgSO_4$ (Toyobo Co., Ltd.), 100 mg of DNA of the strain of the present embodiment, 0.5 μM each of the primers and KOD-Plus-(Toyobo Co., Ltd.) was treated at 94 °C for 2 minutes and then was subjected to 30 cycles consisting of thermal denaturation at 94°C for 30 seconds, annealing at 55°C for 30 seconds and extension at 68°C for 1.5 minutes. Finally, after performing extension at 68°C for 7 minutes, the PCR product was obtained. This PCR product was purified using a QIA quick PCR Purification Kit (Qiagen Inc.), and then the base sequence of 16SrRNA gene was determined. Determination of the base sequence of the gene was carried out using a DNA decoding device (DNA sequencer).

For the base sequence of the strain of the present embodiment, a BLAST homology search was carried out in the National Center for Biotechnology Information (NCBI http://www.ncbi.nlm.nih.gov) which is a database of genes, and as a result, it was found that the base sequence was approximate to 16SrRNA gene of a known bacteria belonging to genus Geobacillus which is registered with the database.

From the results of the test for mycological characteristics such as the above or from the base sequence of 16SrRNA gene, the strain of the present embodiment was acknowledged to be a novel microorganism belonging to genus Geobacillus and was designated as Geobacillus sp. SPT4, which was deposited with the International Patent. Organism Depositary of the National Institute of Advanced Industrial Science and Technology on August 18, 2003 (FERM BP-08452).

As described in the following Examples, this strain Geobacillus sp. SPT4 has a property of producing a sludge solubilizing enzyme having an excellent ability of sludge solubilization. Therefore, this strain can be used in various processes for biological treatment of sludge as described later. That is, the above-mentioned strain can be added to a variety of sludge such as biological sludge including raw sludge and excess sludge that are discharged from sewage treatment processes at sewage treatment plants, night soil treatment plants or the like, or organic sludge that is discharged from production processes or from wastewater treatment processes at food plants, chemical plants or the like, to appropriately solubilize these sludges.

The amount of addition of the strain should be suitably selected according to the content of organic solid matter in the sludge to be treated and other properties and is not particularly limited. However, for example, in the case of a bacterial culture solution incubated in a nutrient broth medium (manufactured by Oxoido Limited) for about 15 hours, it is preferable to add about 0.5 to 3% by volume of the solution to the sludge.

(Embodiment 2)

Another strain among the selected four strains was tested for the mycological characteristics in the manner as described above. The mycological characteristics (morphological characteristics, cultivating characteristics and physiological characteristics) are as follows:

A. Morphological characteristics

(1) Shape and size of cell: rod-shaped cell with a width of 0.8 μm and a length of 2.0 to 4.0 μm, cell extension observed
(2) Presence or absence of motility: present
(3) Presence or absence of spore: present

B. Cultivating characteristics (Nutrient agar plate culture)

(1) Colony morphology: circular, entirely smooth edge, low convex
(2) Color: cream color
(3) Gloss: present

C. Physiological characteristics

(1) Gram staining property: +
(2) Nitrate reduction: -
(3) VP test: -
(4) Indole production: -
(5) Hydrogen sulfide production: -
(6) Hydrolysis of starch: -
(7) Use of citric acid: -
(8) Urease: -
(9) Oxidase: +
(10) Catalase: +
(11) Growth conditions

(a) pH: 6.0 to 8.0
(b) Temperature: 50°C to 65°C

(12) Attitude to oxygen: aerobic
(13) O-F test (Hugh Leifson method): - /-
(14) Production of acid and gas from saccharides D-glucose : acid (+) / gas (-)
(15) Fermentability test

(a) Glycerol: +
(b) L-arabinose: +
(c) D-xylose: +
(d) D-galactose: +
(e) D-glucose: +
(f) D-fructose: +
(g) D-mannose: +
(h) D-mannitol: +
(i) Inositol: -
(j) Sorbitol: -
(k) Maltose: +
(l) Lactose: -
(m) Sucrose: +
(n) Trehalose: +

(16) Other physiological characteristics

(a) β-Galactosidase activity: -
(b) Arginine dihydrolase activity: -
(c) Lysine dicarboxylase activity: -
(d) Tryptophan deaminase activity: -
(e) Acetoin production: -
(f) Gelatinase activity: +
(g) Ornithine dicarboxylase activity: - .

Further, the measurement of pH and temperature for growth of the strain among the above-described physiological characteristics was carried out as follows.

A nutrient agar medium (manufactured by Oxoido Limited) was prepared in which pH was adjusted to the pH under test (6.0, 6.5, 7.0, 7.5 and 8.0) using 0.1 N hydrochloric acid (manufactured by Wako Pure Chemical Industries, Ltd.) and 0.1 N sodium hydroxide (manufactured by Wako Pure Chemical Industries, Ltd.), or either of them. To this medium, the strain of the present embodiment was inoculated, and the amount of cell growth for the strain cultivated at 60 °C for 15 hours was observed to measure the degree of growth qualitatively. The results are presented in the following Table 3.

Table 3 +: positive ±: weakly positive

| pH | Degree of growth |
|---|---|
| 6.0 | + |
| 6.5 | + |
| 7.0 | + |
| 7.5 | + |
| 8.0 | ± |

As obvious from Table 3 above, it was found that the strain of the present embodiment grows at a pH in the range of 6.0 to 8.0.

Next, a nutrient agar medium (manufactured by Oxoido Limited) which was adjusted to pH 6.5 using 0.1 N hydrochloric acid (manufactured by Wako Pure Chemical Industries, Ltd.) and 0.1 N sodium hydroxide (manufactured by Wako Pure Chemical Industries, Ltd.), or either of them, was prepared. To this medium, the strain of the present embodiment was inoculated, and the amount of cell growth of the strain cultivated at the temperatures under test (37°C, 45°C, 50°C, 60°C and 65°C) for 15 hours was observed to measure the degree of growth qualitatively. The results are presented in the following Table 4.

Table 4 +: positive - : negative

| Temperature | Degree of growth |
|---|---|
| 37°C | - |
| 45°C | - |
| 50°C | + |
| 60°C | + |
| 65°C | + |

As obvious from Table 4 above, it was found that the strain of the present embodiment grows at a temperature in the range of 50°C to 65°C.

Furthermore, the base sequence of 16SrRNA gene of this strain was interpreted. The sequence is as described in SEQ ID No. 2 in the sequence listing. In this interpretation, 16SrRNA gene of the strain of the present embodiment was amplified by the PCR method. The PCR method was carried out in the same procedure as in Embodiment 1. The temperature conditions and the reaction cycle were also the same as in Embodiment 1.

The primers used were also the same as in Embodiment 1. Also, determination of the base sequence of the gene was carried out with the same DNA decoding device (DNA sequencer) as in Embodiment 1.

For the base sequence of the strain of the present embodiment, a BLAST homology search was carried out in the same database of genes as in Embodiment 1, and as a result, it was found that the base sequence was approximate to 16SrRNA gene of a known bacteria belonging to genus Geobacillus which is registered with the database.

From the results of the test for mycological characteristics such as the above or from the base sequence of 16SrRNA gene, the strain of the present embodiment was acknowledged to be a novel microorganism belonging to genus Geobacillus and was designated as Geobacillus sp. SPT5, which was deposited with the International Patent Organism Depositary of the National Institute of Advanced Industrial Science and Technology on August 18, 2003 (FERM BP-08453).

This strain Geobacillus sp. SPT5 also has a property of producing a sludge solubilizing enzyme having an excellent ability of sludge solubilization in the same way as Geobacillus sp. SPT4 does, and thus can be used in various processes for biological treatment of sludge as described later.

(Embodiment 3)

Another strain among the selected four strains was tested for the mycological characteristics in the manner as described above. The mycological characteristics (morphological characteristics, cultivating characteristics and physiological characteristics) are as follows:

A. Morphological characteristics

(1) Shape and size of cell: rod-shaped cell with a width of 0.7 to 0.8 μm and a length of 2.0 μm
(2) Presence or absence of motility: present
(3) Presence or absence of spore: present

B. Cultivating characteristics (Nutrient agar plate culture)

(1) Colony morphology: circular, entirely smooth edge, low convex
(2) Color: cream color
(3) Gloss: present

C. Physiological characteristics

(1) Gram staining property: +
(2) Nitrate reduction: -
(3) VP test: -
(4) Indole production: -

(5) Hydrogen sulfide production: -
(6) Hydrolysis of starch: -
(7) Use of citric acid: -
(8) Urease: -
(9) Oxidase: +
(10) Catalase: +
(11) Growth conditions

(a) pH: 6.0 to 8.0
(b) Temperature: 50°C to 65°C

(12) Attitude to oxygen: aerobic
(13) O-F test (Hugh Leifson method) : - /-
(14) Production of acid and gas from saccharides D-glucose : acid (+) /gas (-)
(15) Fermentability test

(a) Glycerol: -
(b) L-arabinose: -
(c) D-xylose: -
(d) D-galactose: -
(e) D-glucose: +
(f) D-fructose: +
(g) D-mannose: +
(h) D-mannitol: -
(i) Inositol: -
(j) Sorbitol: -
(k) Maltose: +
(l) Lactose: -
(m) Sucrose: -
(n) Trehalose: +

(16) Other physiological characteristics

(a) β-Galactosidase activity: -
(b) Arginine dihydrolase activity: -
(c) Lysine dicarboxylase activity: -
(d) Tryptophan deaminase activity: -
(e) Acetoin production: -
(f) Gelatinase activity: +
(g) Ornithine dicarboxylase activity: - .

Further, the measurement of pH and temperature for growth of the strain among the above-described physiological characteristics was carried out as follows.

A nutrient agar medium (manufactured by Oxoido Limited) was prepared in which pH was adjusted to the pH under test (6.0, 6.5, 7.0, 7.5 and 8.0) using 0.1 N hydrochloric acid (manufactured by Wako Pure Chemical Industries, Ltd.) and 0.1 N sodium hydroxide (manufactured by Wako Pure Chemical Industries, Ltd.), or either of them. To this medium, the strain of the present embodiment was inoculated, and the amount of cell growth for the strain cultivated at 60°C for 15 hours was observed to measure the degree of growth qualitatively. The results are presented in the following Table 5.

Table 5 +: positive ±: weakly positive

| pH | Degree of growth |
|---|---|
| 6.0 | + |
| 6.5 | + |
| 7.0 | + |

(continued)

| pH | Degree of growth |
|---|---|
| 7.5 | + |
| 8.0 | ± |

As obvious from Table 5 above, it was found that the strain of the present embodiment grows at a pH in the range of 6.0 to 8.0.

Next, a nutrient agar medium (manufactured by Oxoido Limited) which was adjusted to pH 6.5 using 0.1 N hydrochloric acid (manufactured by Wako Pure Chemical Industries, Ltd.) and 0.1 N sodium hydroxide (manufactured by Wako Pure Chemical Industries, Ltd.), or either of them, was prepared. To this medium, the strain of the present embodiment was inoculated, and the amount of cell growth of the strain cultivated at the temperatures under test (37 °C, 45°C, 50°C, 60°C and 65°C) for 15 hours was observed to measure the degree of growth qualitatively. The results are presented in the following Table 6.

Table 6 +: positive - : negative

| Temperature | Degree of growth |
|---|---|
| 37°C | - |
| 45°C | - |
| 50°C | + |
| 60°C | + |
| 65°C | + |

As obvious from Table 6 above, it was found that the strain of the present embodiment grows at a temperature in the range of 50°C to 65°C.

Furthermore, the base sequence of 16SrRNA gene of this strain was interpreted. The sequence is as described in SEQ ID No. 3 in the sequence listing. In this interpretation, 16SrRNA gene of the strain of the present embodiment was first amplified by the PCR method. The PCR method was carried out in the same procedure as in Embodiment 1. The temperature conditions and the reaction cycle were also the same as in Embodiment 1. The primers used were also the same as in Embodiment 1.

Also, determination of the base sequence of the gene was carried out with the same DNA decoding device (DNA sequencer) as in Embodiment 1.

For the base sequence of the strain of the present embodiment, a BLAST homology search was carried out in the same database of genes as in Embodiment 1, and as a result, it was found that the base sequence was approximate to 16SrRNA gene of a known bacteria belonging to genus Geobacillus which is registered with the database.

From the results of the test for mycological characteristics such as the above or from the base sequence of 16SrRNA gene, the strain of the present embodiment was acknowledged to be a novel microorganism belonging to genus Geobacillus and was designated as Geobacillus sp. SPT6, which was deposited with the International Patent Organism Depositary of the National Institute of Advanced Industrial Science and Technology on August 18, 2003 (FERM BP-08454).

This strain Geobacillus sp. SPT6 also has a property of producing a sludge solubilizing enzyme having an excellent ability of sludge solubilization in the same way as Geobacillus sp. SPT4 does, and thus can be used in various processes for biological treatment of sludge as described later.

(Embodiment 4)

Another strain among the selected four strains was tested for the mycological characteristics in the manner as described above. The mycological characteristics (morphological characteristics, cultivating characteristics and physiological characteristics) are as follows:

A. Morphological characteristics

(1) Shape and size of cell: rod-shaped cell with a width of 0.7 to 0.8 μm and a length of 2.0 to 4.0 μm
(2) Presence or absence of motility: present

(3) Presence or absence of spore: present

B. Cultivating characteristics (Nutrient agar plate culture)

(1) Colony morphology: circular, entirely smooth edge, low convex
(2) Color: cream color
(3) Gloss: present

C. Physiological characteristics

(1) Gram staining property: +
(2) Nitrate (Sulfate) reduction: -
(3) VP test: -
(4) Indole production: -
(5) Hydrogen sulfide production: -
(6) Hydrolysis of starch: +
(7) Use of citric acid: -
(8) Urease: -
(9) Oxidase: +
(10) Catalase: +
(11) Growth conditions

(a) pH: 6.0 to 8.0
(b) Temperature: 45°C to 65°C

(12) Attitude to oxygen: aerobic
(13) O-F test (Hugh Leifson method): - /-
(14) Production of acid and gas from saccharides D-glucose: acid (+)/gas (-)
(15) Fermentability test

(a) Glycerol: -
(b) L-arabinose: +
(c) D-xylose: +
(d) D-galactose: -
(e) D-glucose: +
(f) D-fructose: +
(g) D-mannose: +
(h) D-mannitol: +
(i) Inositol: -
(j) Sorbitol: -
(k) Maltose: +
(l) Lactose: -
(m) Sucrose: -
(n) Trehalose: +

(16) Other physiological characteristics

(a) β-Galactosidase activity: -
(b) Arginine dihydrolase activity: -
(c) Lysine dicarboxylase activity: -
(d) Tryptophan deaminase activity: -
(e) Acetoin production: -
(f) Gelatinase activity: +
(g) Ornithine dicarboxylase activity: - .

Further, the measurement of pH and temperature for growth of the strain among the above-described physiological characteristics was carried out as follows.

A nutrient agar medium (manufactured by Oxoido Limited) was prepared in which pH was adjusted to the pH under test (6.0, 6.5, 7.0, 7.5 and 8.0) using 0.1 N hydrochloric acid (manufactured by Wako Pure Chemical Industries, Ltd.) and 0.1 N sodium hydroxide (manufactured by Wako Pure Chemical Industries, Ltd.), or either of them. To this medium, the strain of the present embodiment was inoculated, and the amount of cell growth for the strain cultivated at 60°C for 15 hours was observed to measure the degree of growth qualitatively. The results are presented in the following Table 7.

Table 7 +: positive ±: weakly positive

| pH | Degree of growth |
|---|---|
| 6.0 | ± |
| 6.5 | + |
| 7.0 | + |
| 7.5 | + |
| 8.0 | + |

As obvious from Table 7 above, it was found that the strain of the present embodiment grows at a pH in the range of 6.0 to 8.0.

Next, a nutrient agar medium (manufactured by Oxoido Limited) which was adjusted to pH 6.5 using 0.1 N hydrochloric acid (manufactured by Wako Pure Chemical Industries, Ltd.) and 0.1 N sodium hydroxide (manufactured by Wako Pure Chemical Industries, Ltd.), or either of them, was prepared. To this medium, the strain of the present embodiment was inoculated, and the amount of cell growth of the strain cultivated at the temperatures under test (37°C, 45°C, 50°C, 60°C and 65°C) for 15 hours was observed to measure the degree of growth qualitatively. The results are presented in the following Table 8.

Table 8 +: positive ±: weakly positive - : negative

| Temperature | Degree of growth |
|---|---|
| 37°C | - |
| 45°C | ± |
| 50°C | + |
| 60°C | + |
| 65°C | + |

As obvious from Table 8 above, it was found that the strain of the present embodiment grows at a temperature in the range of 45°C to 65°C.

Furthermore, the base sequence of 16SrRNA gene of this strain was interpreted. The sequence is as described in SEQ ID No. 4 in the sequence listing. In this interpretation, 16SrRNA gene of the strain of the present embodiment was first amplified by the PCR method. The PCR method was carried out in the same procedure as in Embodiment 1. The temperature conditions and the reaction cycle were also the same as in Embodiment 1.

The primers used were also the same as in Embodiment 1. Also, determination of the base sequence of the gene was carried out with the same DNA decoding device (DNA sequencer) as in Embodiment 1.

For the base sequence of the strain of the present embodiment, a BLAST homology search was carried out in the same database of genes as in Embodiment 1, and as a result, it was found that the base sequence was approximate to 16SrRNA gene of a known bacteria belonging to genus Geobacillus which is registered with the database.

From the results of the test for mycological characteristics such as the above or from the base sequence of 16SrRNA gene, the strain of the present embodiment was acknowledged to be a novel microorganism belonging to genus Geobacillus and was designated as Geobacillus sp. SPT7, which was deposited with the International Patent Organism Depositary of the National Institute of Advanced Industrial Science and Technology on August 18, 2003 (FERM BP-08455).

This strain Geobacillus sp. SPT7 also has a property of producing a sludge solubilizing enzyme having an excellent ability of sludge solubilization in the same way as Geobacillus sp. SPT4 does, and thus can be used in various processes for biological treatment of sludge.

EXAMPLE

Hereinafter, Examples of the present invention will be described.

(Example 1)

The strain of each embodiment described above was used to carry out the test for sludge solubilization.

The test for sludge solubilization was carried out using excess sludge collected from a sewage treatment plant in Kobe. The method of preparing the sterilized, cleaned sludge used in the solubilization test and the method of solubilization test will be explained below.

[Method of preparing sterilized sludge]

1. Excess sludge was subjected to autoclaving at 121°C for 15 minutes and then to centrifugation (about 15,000 g, 10 minutes) to recover the settlement.

2. The settlement was suspended sufficiently in pure water and then subjected to autoclaving and centrifugation under the above-described conditions, to recover the settlement.

3. The settlement was resuspended in pure water and then cleaned by centrifugation. This operation was repeated twice.

4. The settlement was suspended in pure water to a concentration of about 10,000 mg/l and then subjected to autoclaving. The resulting product was supplied to the test.

[Method of solubilization test]

1. Each strain incubated overnight in a LB liquid medium (manufactured by Difco Inc.; 10 g of bactotrypton, 5 g of a yeast extract, 5 g of sodium chloride and 1 L of distilled water) at 63°C was added to the above-described sterilized, cleaned sludge to a volume ratio of 1% and was subjected to shaking incubation at 63°C. The concentration of the volatile suspended solids (VSS) was measured according to the Sewage Testing Method (Vol.1) [Japan Sewage Works Association, pp.296-297 (1997)].

2. The VSS solubilization rate for the sludge was determined based on the following equation:

$$\text{VSS Solubilization Rate} = [(\text{starting VSS concentration} - \text{VSS concentration after incubation})/\text{starting VSS concentration}] \times 100(\%)$$

The test results are presented in Table 9. In Table 9, control means the case where bacteria are not added to the system.

Table 9

| Incubation time (hr) | SPT4 | SPT5 | SPT6 | SPT7 | Control |
|---|---|---|---|---|---|
| 0 | 0 | 0 | 0 | 0 | 0 |
| 24 | 25.7 | 26.3 | 26.1 | 23.5 | 3.6 |
| 48 | 35.5 | 35.0 | 36.7 | 33.2 | 4.5 |

As it is obvious in Table 9, all of the strains of Embodiments 1 to 4 exhibited excellent sludge solubilization rates of approximately 25% after 24 hours of incubation and excellent sludge solubilization rates of approximately 35% after 48 hours of incubation.

Although solubilization was carried out at 63°C as described above in the sludge solubilization test of the present Example, the temperature for solubilization is not limited to this. Solubilization can be carried out at a temperature in the range of 45 to 70°C in accordance with the temperature for growth of the strains of Embodiments 1 to 4. However, in order to attain an excellent solubilization rate, it is preferable to set the temperature in the range of 50 to 65°C.

Next, a mixture of the strain of Embodiment 1, 2 or 3 and the strain of Embodiment 4 was used to carry out a test for comparing the sludge solubilization rate with that of the case where a single bacterium was used. The results are presented in Table 10.

Table 10

| Incubation time (hr) | SPT4 | SPT5 | SPT6 | SPT7 | SPT4+ SPT7 | SPT5+ SPT7 | SPT6+ SPT7 |
|---|---|---|---|---|---|---|---|
| 0 | 0 | 0 | 0 | 0 | 0 | 0 | 0 |
| 24 | 25.3 | 26.8 | 24.4 | 22.7 | 26.6 | 27.7 | 25.5 |
| 48 | 36.0 | 35.4 | 37.1 | 33.8 | 38.0 | 42.5 | 39.3 |

As obvious from Table 10, when a bacterial mixture of the strain of Embodiment 1, 2 or 3 and the strain of Embodiment 4 was used, the sludge solubilization rate increased as compared with the case where a single bacterium was used. This is contemplated to reflect that strain SPT7 of Embodiment 4 grows under higher pH conditions. That is, when proteins in the sludge are first decomposed by enzymes (a protease, a lipase, etc.) produced by those thermophilic bacteria other than strain SPT7 to produce ammonia, thus pH of the sludge being increased. Thus, it is believed that under such conditions, SPT7 proliferates actively and produces enzyme related to sludge solubilization to promote sludge solubilization.

Further, in the case of solubilizing organic solid matter with a mixture of the strain of Embodiment 1, 2 or 3 and the strain of Embodiment 4, it is preferable to treat the solid matter at a pH in the range of 7.0 to 8.5.

(Example 2)

Strains Geobacillus sp. SPT4, Geobacillus sp. SPT5, Geobacillus sp. SPT6 and Geobacillus sp. SPT7 of the respective Embodiments described above have a property of producing a sludge solubilizing enzyme which has an excellent ability of sludge solubilization as described above and thus can be used in various processes for biological treatment of sludge.

In the present Example, an example of the process for biological treatment will be described.

An apparatus for carrying out the process for biological treatment of the present Example is provided with, as shown in Fig. 1, a biological treatment unit, a settling tank and a solubilization tank. In the present Example, wastewater A is introduced into a biological treatment tank 2 via a path 1, and the wastewater, which is organic wastewater, is subjected to aerobic biological treatment at the biological treatment tank 2. Aerobic biological treatment means decomposition of organic matter into inorganic matter such as carbon dioxide or water by biological oxidation.

Next, treated water B which has been biologically treated is introduced into a settling tank 4, which is a unit for solid-liquid separation, via a path 3 and is subjected to solid-liquid separation. Supernatant liquid C resulting from solid-liquid separation is discharged or the like, and a portion of sludge D which is the solid matter resulting from solid-liquid separation passes through a path 5, joins the path 1 and is introduced into the biological treatment tank 2 together with wastewater A.

The remaining portion of sludge E separated at the settling tank 4 is introduced into the solubilization tank 7 via the path 6. At the solubilization tank 7, solubilization of organic solid matter is carried out aerobically under high temperature conditions. In this solubilization treatment, each of the novel microorganisms of the above Embodiments 1 to 4 is used.

The solubilization tank 7 is intended for solubilization of the sludge supplied from the biological treatment tank 2 as described above, and this solubilization process is achieved by solubilizing enzymes such as a protease. Here, such solubilizing enzymes are those produced by the strains of Embodiments 1 to 4, namely, Geobacillus sp. SPT4 [FERM BP-08452], Geobacillus sp. SPT5 [FERM BP-08453], Geobacillus sp. SPT6 [FERM BP-08454] and Geobacillus sp. SPT7 [FERM BP-08455] . These strains may be either preliminarily maintained in the solubilization tank 7, preliminarily included in the sludge supplied to the solubilization tank 7, or freshly added into the solubilization tank 7. Also, these four strains can be used individually or in mixtures.

The temperature of the solubilization tank 7 is set to a temperature in the range of 45 to 70°C, and preferably to a temperature in the range of 50 to 65°C, in accordance with the temperature for growth of the strains of Embodiments 1 to 4. Further, the pH is preferably set to a pH in the range of 5.5 to 9, and preferably to a pH in the range of 6 to 8.5, in accordance with the pH for growth of the strains of Embodiments 1 to 4.

(Example 3)

The present Example is another example of the process for biological treatment, and the treatment apparatus of the present Example is provided with, as shown in Fig. 2, a concentrating unit 9 downstream to the settling tank 4 and upstream to the solubilization tank 7. As sludge is concentrated at the concentrating unit 9 before being supplied to the solubilization tank 7, the amount of introduction of sludge into the solubilization tank is reduced, and consequently HRT at the solubilization tank 7 is extended, thus it being possible to significantly reduce the amount of BOD in the treated liquid that is returned from the solubilization tank 7 to the biological treatment tank 2. For the concentrating unit 9, a concentrating apparatus equipped with means for membrane concentration, centrifugal concentration, floating concentration, evaporative concentration or the like can be used.

The temperature at the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added into the solubilization tank 7.

The treatment apparatus of this Fig. 2 is arranged such that a portion of sludge D which is solid matter resulting from solid-liquid separation passes through the path 5, joins the path 1 and is introduced into the biological treatment tank 2 together with wastewater A, and the remaining portion of sludge E separated at the settling tank 4 is supplied to the concentrating unit 9. However, the arrangement is not limited to this, and it is also possible to concentrate at the concentrating unit 9 all of sludge D that has been separated at the settling tank 4, then to return a portion of the sludge to the biological treatment tank 2 via the path 5, and to solubilize the remaining sludge at the solubilization tank 7 by means of thermophilic bacteria, as shown in Fig. 3.

(Example 4)

In the present Example, as shown in Fig. 4, a membrane separation unit 10, by which solid-liquid separation is carried out in parallel with biological treatment, is disposed inside the biological treatment tank 2. Therefore, solid-liquid separation is more suitably carried out.

In the membrane separation unit 10 that is disposed inside the biological treatment tank 2, for example, a membrane having pore size of from 0.1 to 2.5 μm, preferably from 0.3 to 0.5 μm, is used.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

(Example 5)

The present Example is an example for removing a phosphorus component by microorganisms. A treatment apparatus of the present Example is arranged such that, as shown in Fig. 5, a biological treatment tank 2 includes an anaerobic tank 2a and an aerobic tank 2b. Further, a phosphorus releasing unit 11 is disposed downstream to the settling tank 4 and upstream to the solubilization tank 7. In the present Example, phosphorus is released from microorganisms in the anaerobic tank 2a, and then the aerobic digestion by microorganisms and ingestion of the phosphorus component by microorganisms (accumulation in body) are achieved in the aerobic tank 2b.

Next, the treated liquid that has been biologically treated is separated into primary treated water a and primary sludge x which contains the concentrated phosphorus component, in the settling tank 4. In order to release phosphorus component from the microorganism in primary sludge x, the phosphorus component is released in the liquid phase at the phosphorus releasing unit 11. In this case, release of the phosphorus component can be carried out by, for example, anaerobic treatment, heat treatment, ultrasonic treatment, ozone treatment, alkaline treatment or the like, and it is particularly preferable to carry out it by anaerobic treatment. Next, a flocculating agent is added to secondary treated water b, and the phosphorus component is flocculated as a solid component at a phosphorus separating unit 12 to give tertiary treated water c which substantially does not contain a phosphorus component, and a solid phosphorus component y. This solid phosphorus component y can be used as a raw material for production of fertilizers or phosphorus compounds. The foregoing secondary sludge z is further subjected to solubilization at the solubilization tank 7 for volume reduction of the sludge fraction.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8 . 5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

(Example 6)

The present Example is an example for the case where the loss of heat energy is small, the content of the nitrogen-containing organic components or the nitrogen-containing inorganic components in the treated water that is discharged out of the treatment system is small, and deodorization of an exhaust gas emitted to the atmosphere is possible. As shown in Fig. 6, on the route of the treated liquid to the biological treatment tank 2, a nitrification unit 13 and a denitrification unit 14 are disposed, andaportion of the sludge separated at the settling tank 4 is returned to the nitrification unit 13 via a recycle path 15, while the treated liquid solubilized at the solubilization tank 7 is returned to the denitrification unit 14 via a heat exchanger 16 and a return path 17. Also, air which has entered the solubilization tank 7 via the path 26 is sent to the nitrification unit 13 via the path 27.

The $NH_4^+$ component in the organic wastewater is changed to $NO_2^-$ or $NO_3^-$ by nitrifying bacteria at the nitrification unit 13, and this $NO_2^-$ or $NO_3^-$ is introduced to the denitrification unit 14 and then emitted to the atmosphere, thus the odor of the gas emitted to the atmosphere being significantly attenuated. Further, the heat of the gas discharged from the solubilization tank 7 is effectively used in the nitrification treatment at the nitrification unit 13, and thus the loss of heat energy is small.

Furthermore, the content of the nitrogen-containing organic components or the nitrogen-containing inorganic components in the treated water discharged out of the treatment system is substantially zero.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

(Example 7)

The apparatus for treatment of organic wastewater of the present Example includes a biological treatment tank 2 and a solubilization tank 7, as shown in Fig. 7. In the biological treatment tank 2, treatment of organic wastewater is achieved batchwise. In the present Example, sewage water was used as the organic wastewater.

In the present Example, treatment is achieved by carrying out the processes of inflow of raw water, reaction, settling, draining, sludge discharge and the like in one cycle. More specifically, as shown in Fig. 8, while the system receives an inflow of raw water, the processes of aeration, stirring, aeration, stirring, aeration, settling due to stopped aeration, solid-liquid separation and solubilization treatment are carried out in cycles. In this case, aeration is an aerobic treatment, while stirring is an anaerobic treatment. Repeated processes of aeration and stirring, and the processes of settling and solid-liquid separation are carried out in the biological treatment tank 2, and the process of solubilization treatment is carried out in the solubilization tank 7.

A series of the batch processes for wastewater treatment from the reception of an inflow of raw water to the discharge of treated water can allow adjustment of the duration of treatment in each process so that the processes are carried out multiple times (e.g., 2 to 4 times) a day; however, depending on the nature and state of wastewater, the duration of treatment in each process may be adjusted such that the batch process is carried out, for example, about once a day or twice per three days.

In the present Example, the process of aeration involves nitrification by nitrifying bacteria, and the process of stirring with aeration being stopped involves denitrification by denitrifying bacteria. Then, upon stopping of aeration, sludge is settled and separated. The supernatant is discharged or the like, and a portion of the settled sludge is retained in the biological treatment tank 2 for the subsequent batch process, while the remaining portion of the sludge is supplied to the solubilization tank 7 for solubilization treatment. The liquid treated by solubilization at the solubilization tank 7 is preferably returned to the process of stirring at the first step, as shown in Fig. 9.

The solubilization treated liquid is returned to the biological treatment tank 2 in 3 hours to 30 minutes, and preferably in 1 hour to 30 minutes, before the stopping of aeration at the first step. The number of cycles is determined by the BOD-SS load at the biological treatment tank. In general, in the case of high load operation (BOD-SS load: 0.2 to 0.4 kg BOD/kg SS·day), it is preferable that the nitrification-denitrification treatment with aeration and stirring is operated in 3 or 4 cycles. Also, in the case of low load operation (BOD-SS load: 0.03 to 0.05 kg BOD/kg SS·day), it is preferable that the nitrification-denitrification treatment is operated in 2 or 3 cycles.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

In the present Example, the solubilized sludge is returned to the reaction tank of the first (initial) aeration process in 3 hours to 30 minutes, and preferably in 1 hour to 30 minutes, before stopping of the aeration. It is possible thereby to effectively use the organic matter contained in the solubilized sludge as a source of proton (source of BOD) during the denitrification treatment in order to promote denitrification. Therefore, since the amount of chemical agents such as methanol that are generally used as the source of proton can be reduced, the cost associated with the amount of chemical agents can be also reduced.

In this case, the duration for solubilization treatment is preferably from 12 to 72 hours, more preferably from 18 to 48 hours, and most preferably from 20 to 36 hours.

(Example 8)

The apparatus for treatment of organic wastewater of the present embodiment is provided with an anaerobic tank 18, a primary aeration tank 19, an anoxic tank 20, a secondary aeration tank 21, a settling tank 4 and a solubilization tank 7 as shown in Fig. 9.

An anaerobic tank 18 has a function of anaerobically digesting organic wastewater, and in the case where the return sludge or the sludge in the acid-fermented liquid contains phosphorus, the function of releasing the phosphorus in the sludge into the liquid.

A primary aeration tank 19 is intended for aerobic biological treatment through aeration and stirring of the treated liquid that has been anaerobically treated at the foregoing anaerobic tank 18, and for oxidative decomposition of the organic matter in anaerobically treated water, or nitrification of the ammonia inflow. This primary aeration tank 19 may be any one which is notably equipped with an aeration means, and for the aeration means, though not limited, for example, an aeration tube can be used. The aeration treatment is carried out preferably at room temperature and at a ventilation volume of 0.1 to 0.5 vvm in order to allow aerobic digestive decomposition; however, depending on the load, the treatment can be carried out at a higher ventilation volume and a higher temperature. The liquid to be treated is adjusted preferably to pH 5.0 to 8.0, and more preferably to pH 7.0 to 8.0.

An anoxic tank 20 is intended for denitrification of the treated liquid that has been aerobically treated at the foregoing primary aeration tank 19.

A secondary aeration tank 21 is intended to subject the treated liquid that has been denitrified at the foregoing anoxic tank 20 to aerobic biological treatment. This secondary aeration tank 21 has the same arrangement as the foregoing primary aeration tank 19, and biological treatment is carried out by aeration and stirring in the same manner. In this case, the secondary aeration tank 21 has both functions of nitrification and BOD removal. Further, although not shown in the figure, a portion of the nitrified liquid which is the treated liquid from secondary aeration tank 21, is returned to the anoxic tank 20 for denitrification of nitric acid or nitrous acid in the nitrified liquid.

The settling tank 4 is intended for solid-liquid separation of the treated liquid that has been biologically treated at the foregoing secondary aeration tank 21, and the separated liquid fraction is recycled as a treated liquid or discharged, while the sludge which is the separated and settled solid fraction is then partly supplied to the solubilization tank 7, with the remaining portion being returned to the anaerobic tank 18.

When sewage water is treated in a treatment apparatus having such an arrangement, sewage water is first supplied to the anaerobic tank 18. The treated water after anaerobic treatment is supplied to the primary aeration tank 19 of the next process and treated aerobically by aeration and stirring. This aerobic treatment by aeration and stirring leads to the nitrification treatment.

Next, the treated liquid that has been treated by aeration at the primary aeration tank 19 is supplied to the anoxic tank 20. In this anoxic tank 20, denitrification is carried out. The treated liquid that has been denitrified at the anoxic tank 20 is supplied to the secondary aeration tank 21 and treated aerobically by aeration and stirring. This aeration at the secondary aeration tank 21 leads to nitrification and removal of BOD.

Next, the treated liquid that has been aerated at the secondary aeration tank 21 is supplied to the settling tank 4. In this settling tank 4, solid-liquid separation occurs, and the separated liquid fraction is recycled as a treated liquid or discharged, while the sludge which is the separated and settled solid fraction is partly supplied to the solubilization tank 7 so that the sludge is solubilized aerobically by the strain of the above-described embodiment. Also, the remaining portion of the settled sludge is returned to the anaerobic tank 18 as a return sludge.

The sludge solubilized at the solubilization tank 7 is returned to the aforementioned anoxic tank 20 and treated again. Then, denitrification at the anoxic tank 20, aeration at the secondary aeration tank 21, solid-liquid separation at the settling tank 4 and solubilization at the solubilization tank 7 are repeated in cycles.

HRT at the solubilization tank 7 is preferably selected based on the HRT value which maximizes the amounts of bacterial production and secretion. If HRT is selected in this way, the reaction under the action of the sludge solubilizing enzyme produced and secreted can be efficiently utilized. Typically, HRT is preferably set to 12 to 72 hours, more preferably set to 24 to 72 hours in view of oxidation of ammonia in the solubilized liquid, and most preferably set to 36 to 48 hours in view of balancing between compacting of the solubilization apparatus and improvement of the quality of treated water.

Furthermore, the HRT values at tanks other than solubilization tank 7 are such that 0.5 to 1.5 hours at the anaerobic tank 18, 2 to 6 hours at the primary aeration tank 19, 0.5 to 3 hours at the anoxic tank 20, and 0.5 to 2 hours at the secondary aeration tank 21; and preferably 0.5 to 1 hour at the anaerobic tank 18, 3 to 5 hours at the primary aeration tank 19, 1 to 2 hours at the anoxic tank 20, and 0.5 to 1.5 hours at the secondary aeration tank 21.

The temperature of the solubilization tank 7 is preferably set to a temperature in the range of 45 to 70 °C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

(Example 9)

The present Example is different from the foregoing Example 8 in that there are provided two anoxic tanks and only one aeration tank.

Thus, the treatment apparatus of the present Example is provided with a pre-anoxic tank 23, an anaerobic tank 18, a compatible tank 24, an anoxic tank 20, an aeration tank 25, a settling tank 4, a concentrator 9 and a solubilization tank 7 as shown in Fig. 10.

In the present embodiment, the raw water entering anaerobic tank 18 is supplied to the compatible tank 24.

This compatible tank 24 has a function of changing the return path of the sludge and treated liquid (nitrified liquid) from the aeration tank 25 in accordance with the degree of denitrification of the sewage inflow. For example, during the season of high degree of denitrification such as summer, the compatible tank is used as an anaerobic tank to promote the reaction of releasing phosphorus from the return sludge in the anaerobic state, whereas during the season of low degree of denitrification such as winter, it is used as an anoxic tank to promote denitrification reaction of raw water and the nitrified liquid returned from the aeration tank 25 to the pre-anoxic tank 23 or the compatible tank 24.

After the treatment at the compatible tank 24 as such, raw water is supplied to the anoxic tank 20 for denitrification thereof, and is supplied again to the aeration tank 25 for aerobic biological treatment by aeration and stirring. Next, it is supplied from the aeration tank 25 to the settling tank 4, and is subjected to solid-liquid separation at this settling tank 4, the separated liquid fraction being appropriately discharged. Further, the sludge which is the separated and settled solid fraction is supplied to the concentrator 9 and to the solubilization tank 7. In this case, the aeration tank 25 has the functions of BOD removal and nitrification. A portion of the nitrified liquid which is the treated liquid from the aeration tank 25 is returned to the pre-anoxic tank 23, a nd preferably (though not shown in the figure) to the anoxic tank 20.

The sludge solubilized at the solubilization tank 7 is returned to the compatible tank 24 and is circulated through the cycle of the compatible tank 24, the anoxic tank 20, the aeration tank 25, the settling tank 4, the concentrator 9 and the solubilization tank 7. In addition to being supplied to the concentrator 9, the sludge separated at the settling tank 4 is also returned to the pre-anoxic tank 23. Further, sludge from the anaerobic tank 18 or the compatible tank 24 is also returned to the pre-anoxic tank 23.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

(Example 10)

The treatment apparatus of the present Example is provided with an anaerobic tank 18, an anoxic tank 20, an aeration tank 25, a settling tank 4 and a solubilization tank 7, as shown in Fig. 11.

In the present example, raw water is anaerobically treated at the anaerobic tank 18 so that the phosphorus component in the sludge is released, and is supplied to the anoxic tank 20 where it is subjected to denitrification. The treated liquid which has been denitrified at the anoxic tank 20 is supplied to the aeration tank 25, and ammonia contained in the sludge is changed to nitrous acid or nitric acid at the aeration tank 25. That is, nitrification occurs at the aeration tank 25.

Next, the treated liquid that has been nitrified at the aeration tank 25 is supplied to the settling tank 4. Solid-liquid separation occurs at this settling tank 4, and the separated liquid fraction is discharged or the like. A portion of the sludge which is the separated and settled solid fraction is supplied to the solubilization tank 7, and the remaining portion is returned to the anaerobic tank 18 as return sludge.

The sludge after solubilization treatment is returned to the anaerobic tank 18 and is subjected to repetition of the cycle of denitrification at the anoxic tank 20, treatment at the aeration tank 25, solid-liquid separation at the settling tank 4 and solubilization at the solubilization tank 7. Further, a portion of the nitrified liquid which has been treated at the aeration tank 25 is returned to the anoxic tank 20 or the anaerobic tank 18 and denitrified at the anoxic tank 20.

The temperature of the solubilization tank 7 is set to a temperature in the range of 45 to 70°C, and preferably to a temperature in the range of 50 to 65°C, as in Example 1 above. Further, the pH is also set to a pH in the range of 5.5 to 9, and preferably to a pH in the range of 6 to 8.5, as in Example 2. It is also the same as in Example 2 that the four strains can be used individually or in mixtures. It is also the same as in Example 2 that the strains may be either preliminarily maintained in the solubilization tank 7, preliminarily included in the sludge supplied to the solubilization tank 7, or freshly added to the solubilization tank 7.

## List of reference numbers

**[0143]**

| | |
|---|---|
| A | waste water |
| B | treated water |
| C | supernatant liquid |
| D | sludge |
| E | remaining portion of sludge |
| 1 | path |
| 2 | biological treatment tank |
| 3 | path |
| 4 | settling tank |
| 5 | path |
| 6 | path |
| 7 | solubilization tank |
| 9 | concentrating unit |
| 10 | membrane separation unit |
| a | primary treated water |
| b | secondary treated water |
| c | tertiary treated water |
| x | primary sludge |
| y | solid phosphorus component |
| z | secondary sludge |
| 2a | anaerobic tank |
| 2b | aerobic tank |
| 11 | phosphorus releasing unit |
| 12 | phosphorus separating unit |
| 13 | nitrification unit |
| 14 | denitrification unit |
| 15 | path |
| 15 | heat exchanger |
| 17 | return path |
| 18 | anaerobic tank |

19 primary aeration tank
20 anoxic tank
21 secondary aeration tank
23 pre-anoxic tank
24 compatible tank
25 aeration tank
26 path
27 path

## Claims

1. A novel microorganism is any one of Geobacillus sp. SPT4 (FERM BP-08452), Geobacillus sp. SPT5 (FERM BP-08453), Geobacillus sp. SPT6 (FERM BP-08454) and Geobacillus sp. SPT7 (FERM BP-08455).

2. A process for treatment of organic solid, in which organic solid matter such as organic sludge or biological sludge is solubilized by using a novel microorganism of at least one of Geobacillus sp. SPT4 (FERM BP-08452), Geobacillus sp. SPT5 (FERM BP-08453), Geobacillus sp. SPT6 (FERM BP-08454) and Geobacillus sp. SPT7 (FERM BP-08455).

3. A process for treatment of organic solid according to claim 2, in which organic solid matter such as organic sludge or biological sludge is solubilized by using a mixture of novel microorganisms of any one of Geobacillus sp. SPT4 (FERM BP-08452), Geobacillus sp. SPT5 (FERM BP-08453), or Geobacillus sp. SPT6 (FERM BP-08454), and a microorganism of Geobacillus sp. SPT7 (FERM BP-08455).

## Patentansprüche

1. Neuer Mikroorganismus, welcher aus der Gruppe von Geobacillus sp. SPT4 (FERM BP-08452), Geobacillus sp. SPT5 (FERM BP-08453), Geobacillus sp. SPT6 (FERM BP-08454) und Geobacillus sp. SPT7 (FERM BP-08455) ausgewählt ist.

**2.** Verfahren zur Behandlung organischer Feststoffe, bei welchem organisches Feststoff-Material wie organischer Schlamm oder biologischer Schlamm durch Verwendung eines neuen Mikroorganismus, welcher mindestens einer aus der Gruppe von Geobacillus sp. SPT4 (FERM BP-08452), Geobacillus sp. SPT5 (FERM BP-08453), Geobacillus sp. SPT6 (FERM BP-08454) und Geobacillus sp. SPT7 (FERM BP-08455) ist, aufgelöst wird.

**3.** Verfahren zur Behandlung organischer Feststoffe gemäß Anspruch 2, bei welchem organisches Feststoff-Material wie organischer Schlamm oder biologischer Schlamm durch Verwendung einer Mischung neuer Mikroorganismen, welche aus der Gruppe von Geobacillus sp. SPT4 (FERM BP-08452), Geobacillus sp. SPT5 (FERM BP-08453) oder Geobacillus sp. SPT6 (FERM BP-08454) und eines Mikroorganismus Geobacillus sp. SPT7 (FERM BP-08455) ausgewählt sind, aufgelöst wird.

**Revendications**

**1.** Nouveau microorganisme étant l'un de Geobacillus sp. SPT4 (FERM BP-08452), Geobacillus sp. SPT5 (FERM BP-08453), Geobacillus sp. SPT6 (FERM BP-08454) et Geobacillus sp. SPT7 (FERM BP-08455).

**2.** Procédé de traitement d'un solide organique, dans lequel une matière solide organique telle que la boue organique ou la boue biologique est solubilisée en utilisant un nouveau microorganisme d'au moins l'un de Geobacillus sp. SPT4 (FERM BP-08452), Geobacillus sp. SPT5 (FERM BP-08453), Geobacillus sp. SPT6 (FERM BP-08454) et Geobacillus sp. SPT7 (FERM BP-08455).

**3.** Procédé de traitement d'un solide organique selon la revendication 2, dans lequel une matière solide organique telle que la boue organique ou la boue biologique est solubilisée en utilisant un mélange de nouveaux microorganismes de l'un quelconque de Geobacillus sp. SPT4 (FERM BP-08452), Geobacillus sp. SPT5 (FERM BP-08453), Geobacillus sp. SPT6 (FERM BP-08454) et Geobacillus sp. SPT7 (FERM BP-08455).

Fig . 1

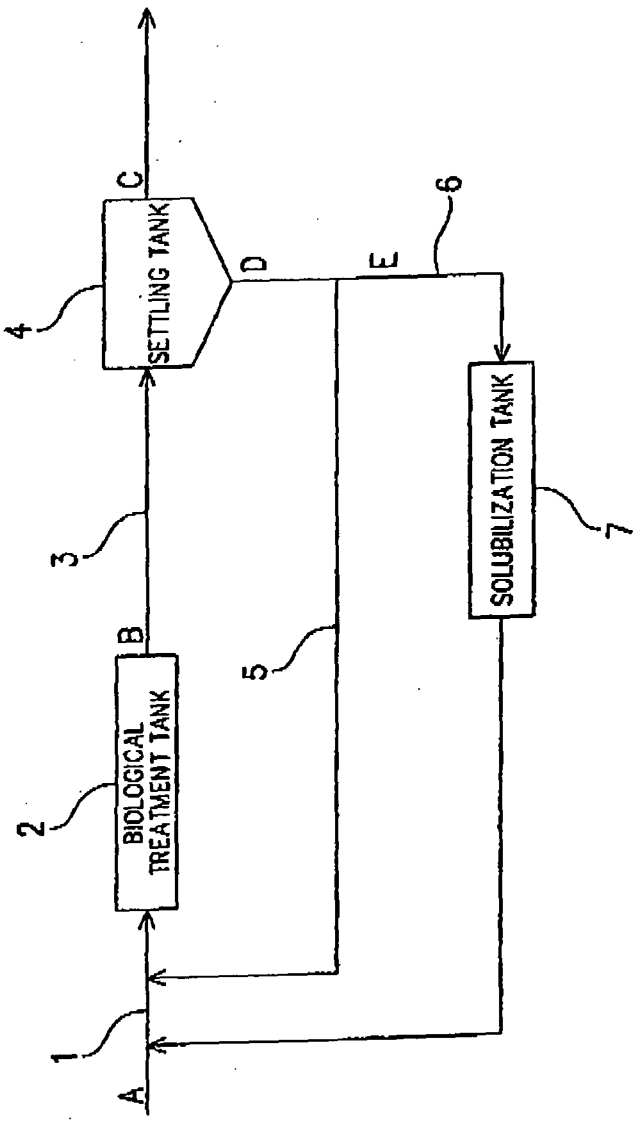
A
1
2
BIOLOGICAL TREATMENT TANK
B
3
4
SETTLING TANK
C
D
E
5
6
SOLUBILIZATION TANK
7

Fig. 2

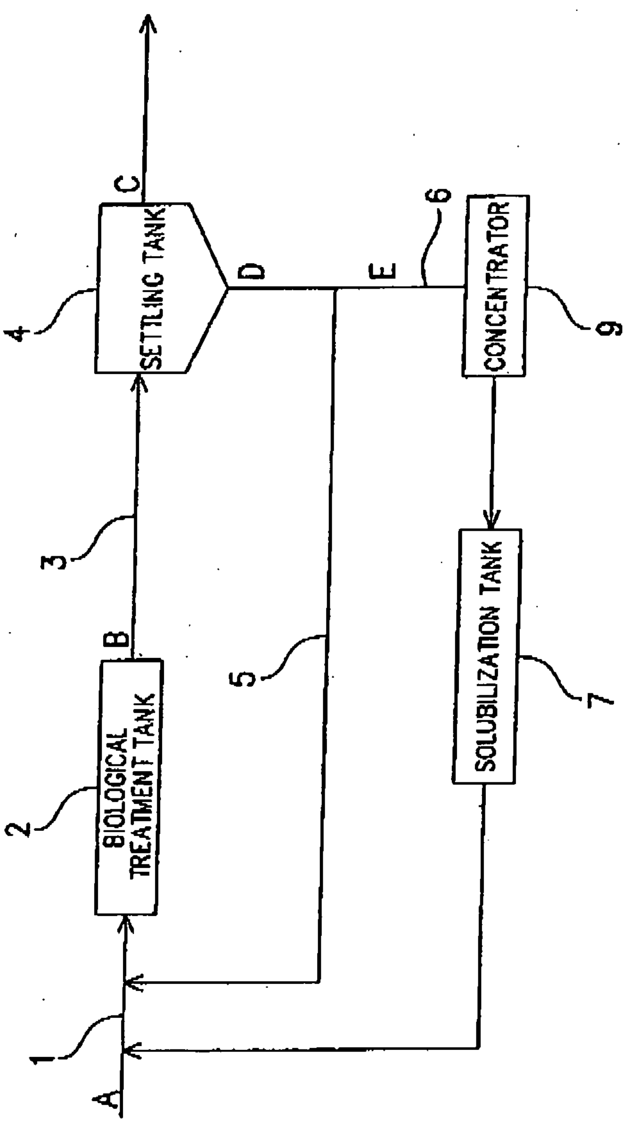
A
1
2
BIOLOGICAL TREATMENT TANK
B
3
4
SETTLING TANK
C
D
E
5
6
CONCENTRATOR
9
SOLUBILIZATION TANK
7

Fig. 3

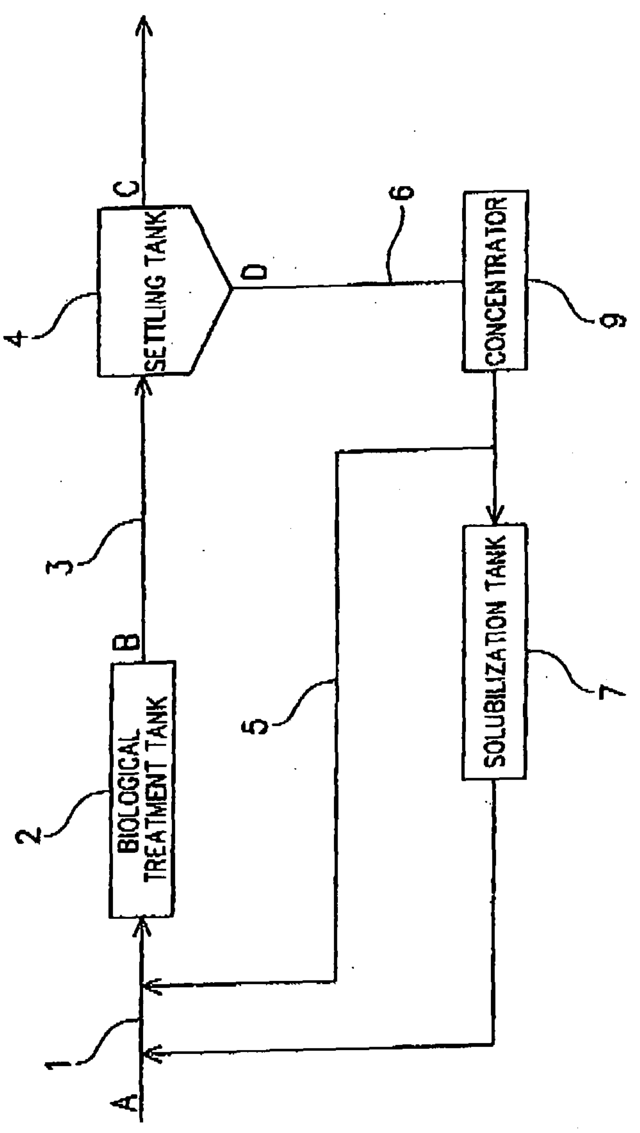
A
1
2
BIOLOGICAL TREATMENT TANK
B
3
4
SETTLING TANK
C
D
6
CONCENTRATOR
9
5
SOLUBILIZATION TANK
7

Fig. 4

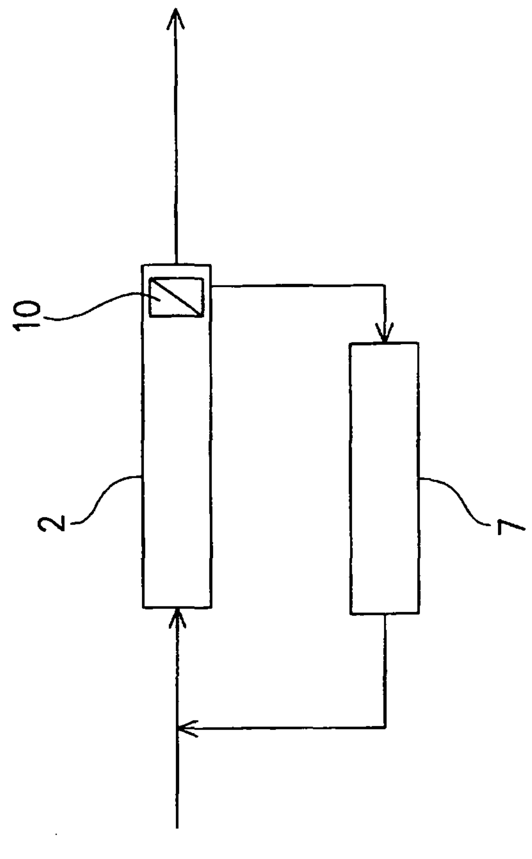

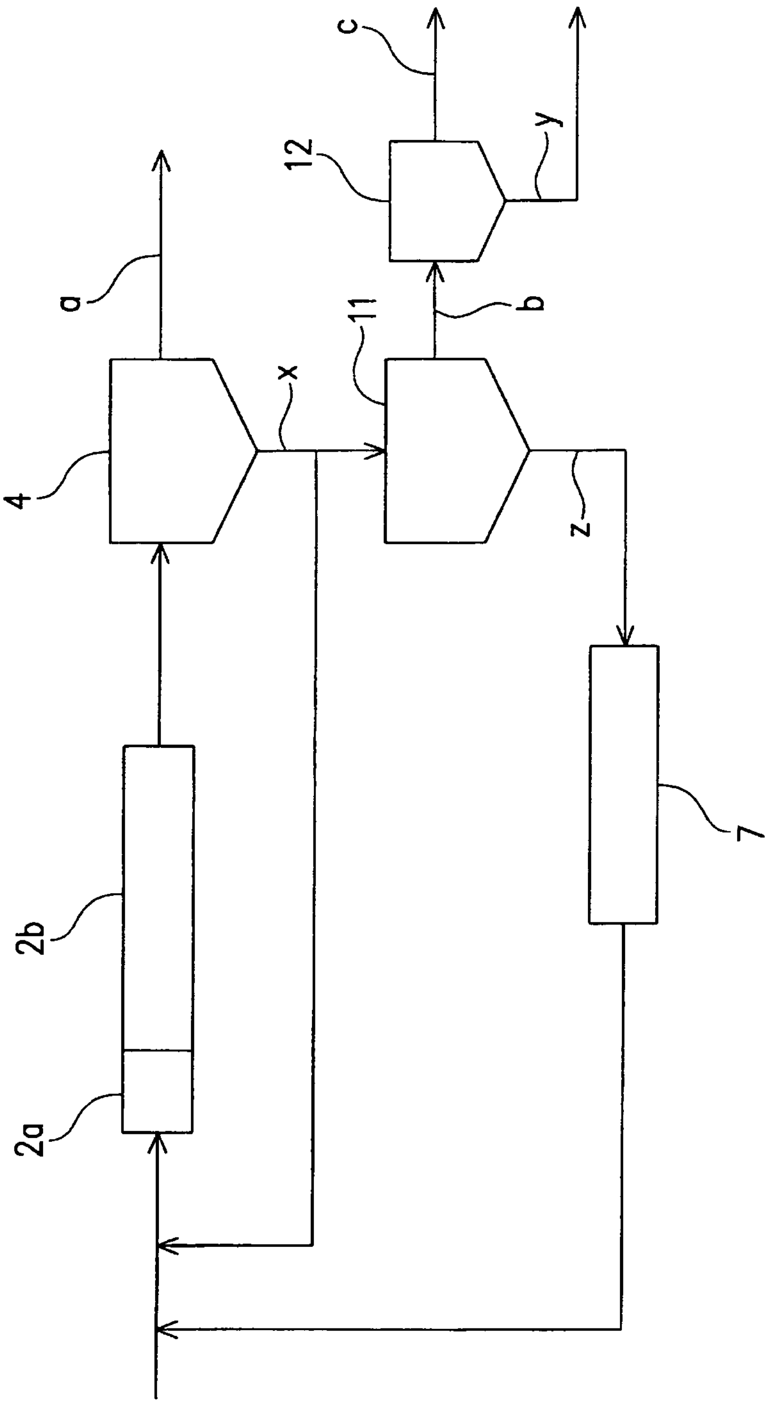


Fig. 5

Fig. 6

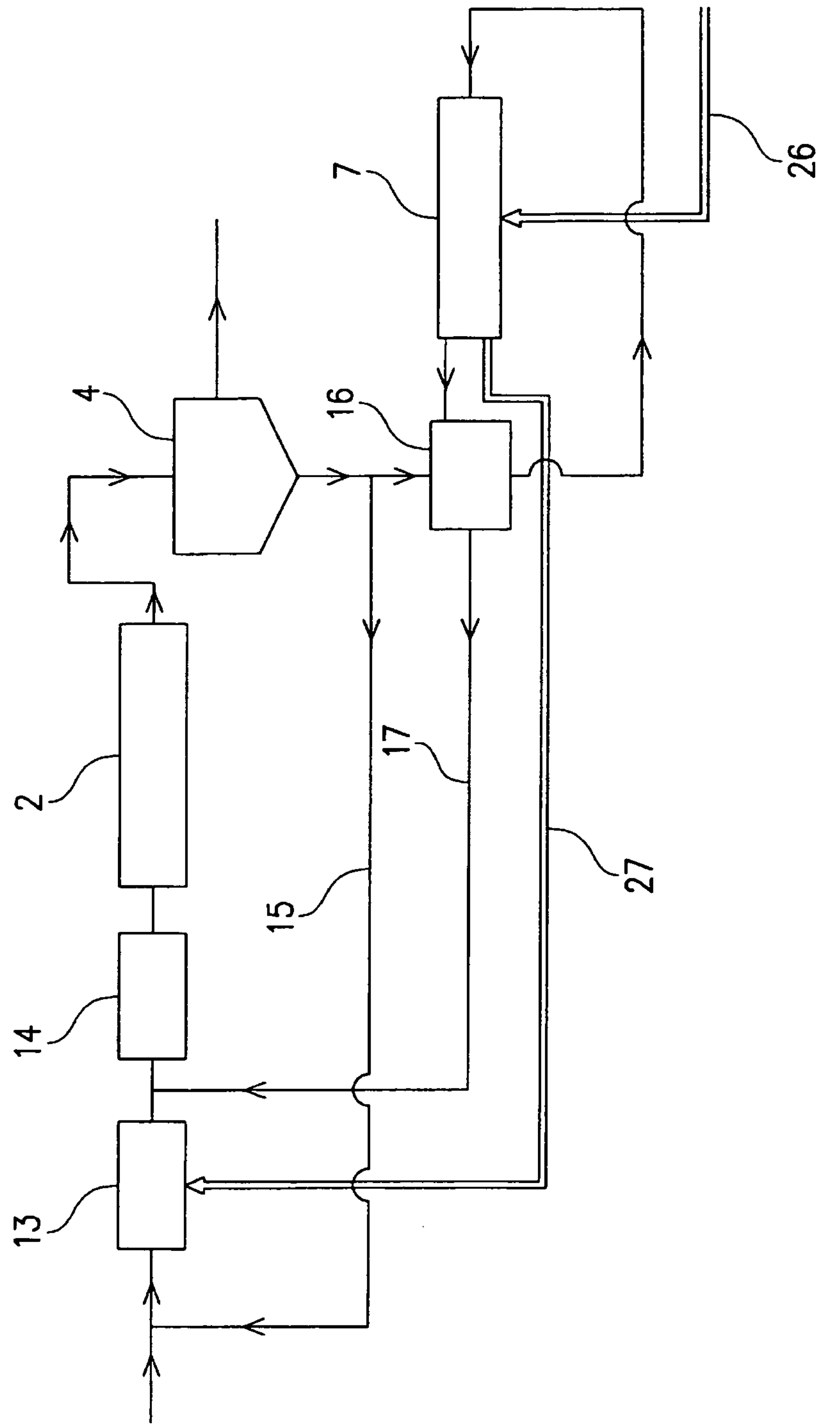

Fig. 7

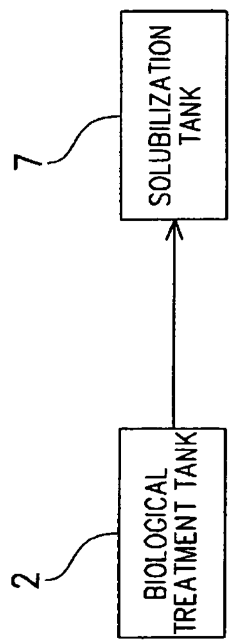

Fig. 8

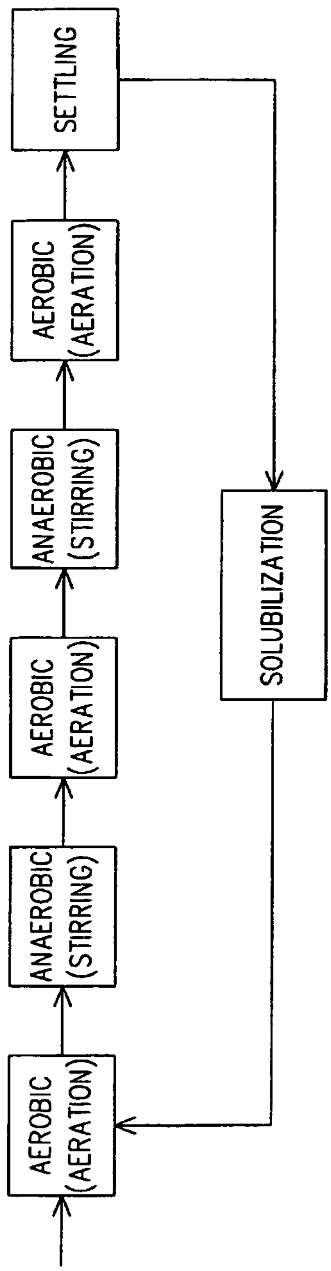
AEROBIC (AERATION)
ANAEROBIC (STIRRING)
AEROBIC (AERATION)
ANAEROBIC (STIRRING)
AEROBIC (AERATION)
SETTLING
SOLUBILIZATION

Fig . 9

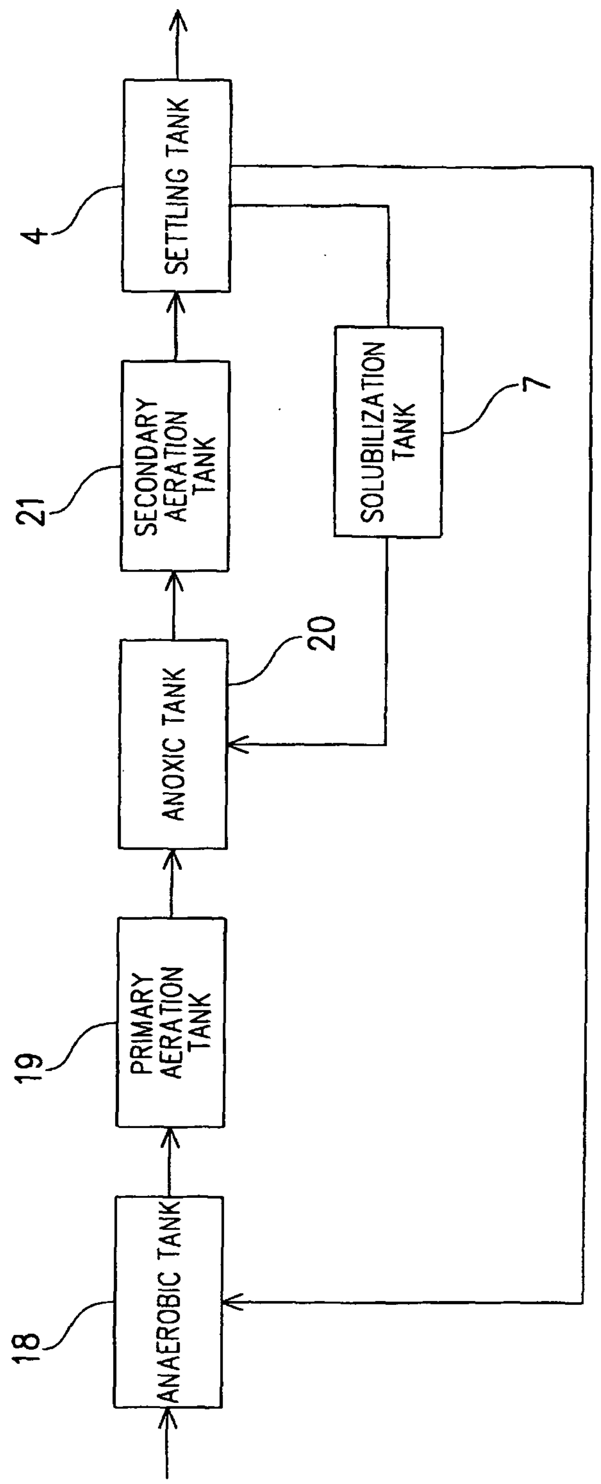
ANAEROBIC TANK
18
PRIMARY AERATION TANK
19
ANOXIC TANK
20
SECONDARY AERATION TANK
21
SETTLING TANK
4
SOLUBILIZATION TANK
7

Fig .10

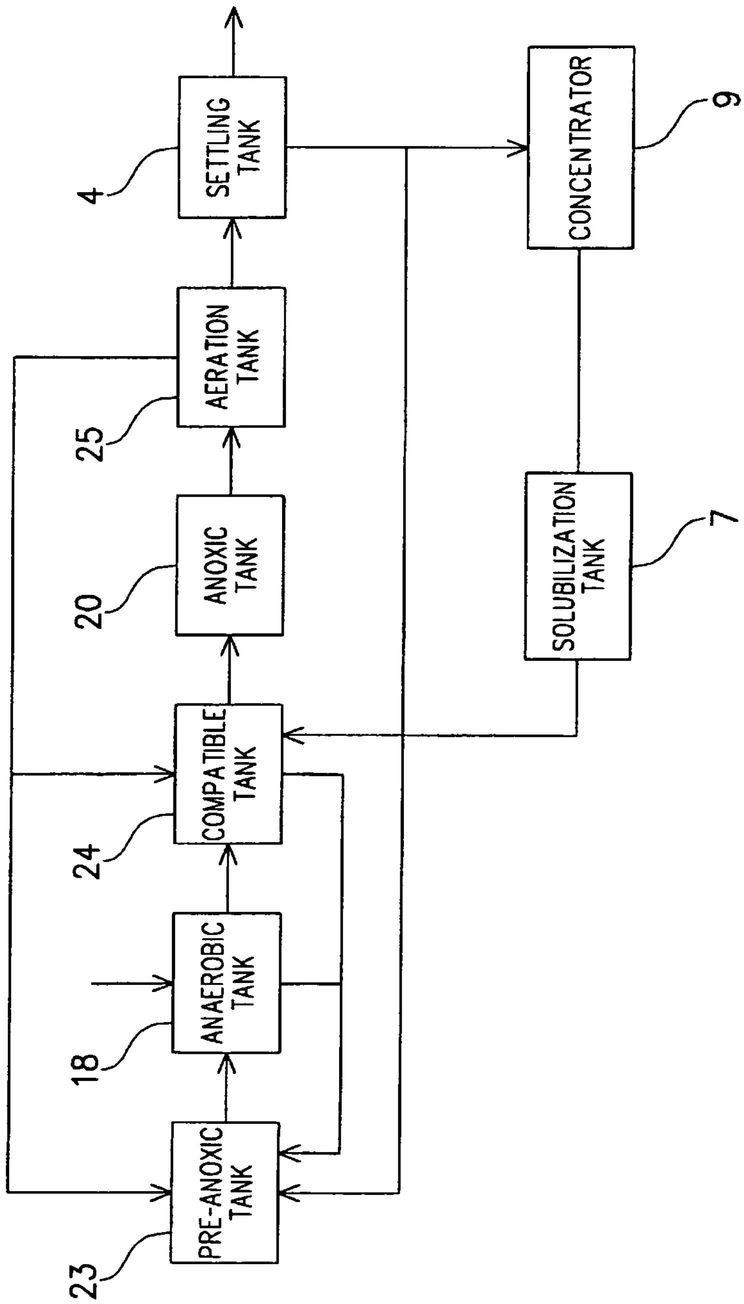

SETTLING TANK
4
AERATION TANK
25
ANOXIC TANK
20
COMPATIBLE TANK
24
ANAEROBIC TANK
18
PRE-ANOXIC TANK
23
CONCENTRATOR
9
SOLUBILIZATION TANK
7

Fig . 11

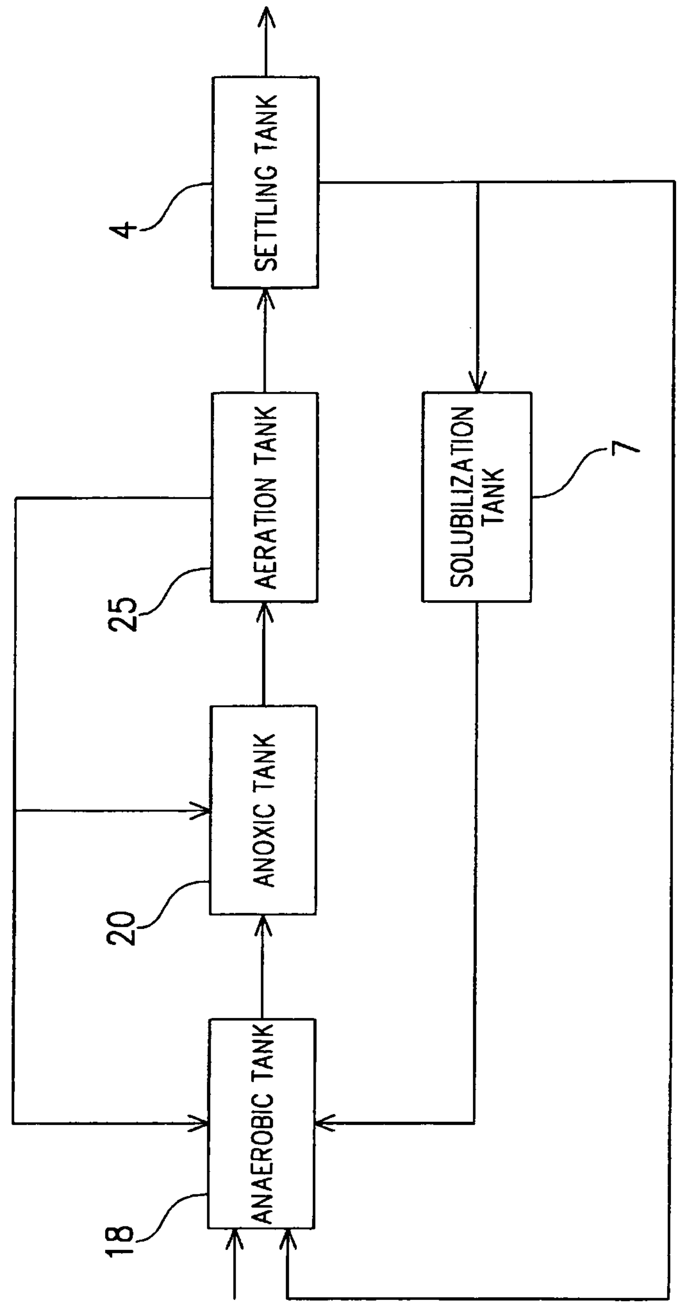
SETTLING TANK
4
AERATION TANK
25
SOLUBILIZATION TANK
7
ANOXIC TANK
20
ANAEROBIC TANK
18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7184640 A
- EP 03818540 A
- JP 2003209106 B


### Non-patent literature cited in the description


- Report on Development of New Drainage Treatment System Utilizing Biotechnology (Part on Sewage. *Public Works Research Center (Japan,* February 1991, 73-77
- **SHIGERU KUME ; YUSAKU FUJIO.** Digestion of Municipal Sewage Sludge by a Mixture of Thermophilic Bacilli and Their Culture Extract. *J. Gen. Appl. Microbiol.,* 1990, vol. 36, 189-194
- **YUSAKU FUJIO ; SHIGERU KUME.** Isolation and Identification of Thermophilic Bacteria from Sewage Sludge Compost. *Journal of Fermentation and Bioengineering,* 1991, vol. 72 (5), 334-337
- **R. BEAUDET ; C. GAGNON ; J.G. BISAILLON ; M. ISHAQUE.** Microbiological Aspects of Aerobic Thermophilic Treatment of Swine waste. *Applied and Environmental Microbiology,* April 1990, 971-976
- **HENG ZHU ; FENG QU ; LI-HUSANG ZHU.** *Isolation of genomic DNAs,* 1993
- Sewage Testing Method. Japan Sewage Works Association, 1997, vol. 1, 296-297